# EUROPEAN PATENT APPLICATION

(11) **EP 2 862 563 A1**
(43) Date of publication of application: **22.04.2015**
(21) Application number: 14182338.5
(22) Date of filing: 18.06.2012
(51) Int. Cl.: A61K 8/40, A61K 8/41, A61Q 17/00, A61Q 19/10

(54) **Foaming topical antimicrobial cleaning compositions**

(30) Priority: 20.06.2011 US 201161498667 P
(62) Divisional of application: 12728759.7
(71) Applicant: Reckitt & Colman (Overseas) Limited, Slough Berkshire SL1 3UH (GB)
(72) Inventor: De Szalay, Sarah Frances, West Milford, NJ New Jersey NJ 07480 (US)
(74) Representative: Bowers, Craig Malcolm

(57) **Abstract**

Provided are largely aqueous topical antimicrobial cleaning composition which provides an antimicrobial benefit, as well as methods for their production and methods for their use. The compositions comprise at least two different cationic surfactants which provide a primary antimicrobial benefit, and as a predominant further surfactant an amine oxide surfactant. The composition are at an acidic pH and are highly effective in providing an antimicrobial benefit to topical surfaces, e.g, epidermis and hair.

## Description

The present invention relates to topical compositions for use in handwash and bodywash applications. More specifically the present invention relates to foaming topical antimicrobial cleansing compositions, methods relating to their use, and methods of their manufacture.

Topical compositions, per se, are well-known in the cosmetic, dermatological as well as in the pharmaceutical fields. Most topical compositions are intended to provide at least one but generally provide multiple or more specific benefits after being applied to the human skin. For example, personal care compositions which are primarily intended to be soaps for general cleaning of the human skin such as hand soaps or body wash soaps are well known in the fields of cosmetics and personal care products. While providing a primary cleaning benefit, such personal care compositions frequently also provide ancillary benefits such as moisturizing and nourishing the skin. Such personal care compositions which provide a good general cleaning benefit are usually based on one or more anionic soaps or anionic surfactants which are recognized to provide good cleaning and good foaming. However, such compositions typically provide only limited germicidal benefits.

Also known to the art are topical compositions which are primarily directed to provide a germicidal benefit to the epidermis or other body part when applied thereto. Such typically take the form of viscous gels and are often largely comprised of an alcohol, usually ethanol, with further constituents, e.g., thickeners. While often technically effective to provide a germicidal benefit, such compositions are also not without shortcomings, including in some cases, an unpleasant skin feel and in other cases, an undesired drying effect to the skin.

For example, cleaning compositions are known from WO 99/19438. The compositions may be used in the cleaning of hard surfaces as well as topical surfaces, e.g. as a hand soap which hand soaps include a nonionic surfactant base in combination with at least one cationic ammonium compound, which latter of which may be an antimicrobial compound and/or a conditioning agent. The compositions necessarily include a nonionic linear alcohol ethoxylate surfactant having an average carbon chain length of not more than 12 carbon atoms, with at least one further surfactant selected from betaine surfacants and amine oxide surfactants. The demonstrated examples of hand soap and conditioning shampoo formulations (at page 42 of the document) provide formulations which include 1%wt. or more of benzalkonium chloride as necessarily being present.

US 2009/318322 discloses viscous compositions having reduced eye irritancy comprising a quaternary ammonium cationic surfactant, an alkamine oxide, a nonionic material, an preferably a thickener constituent selected from nonionic polymer thickeners or cationic polymer thickeners. It is preferred that the alkamine oxide and the nonionic material are present in a ratio which yields a specific Permeabiilty Value of less than about 1.2 in a Bovine Corneal Opacity and Permeability assay. A review of the demonstrated examples indicates that in addition to a thickener constituent (Natrosol 250HHR CS), benzethonium chloride is present at 1%wt., and while lauramine oxide is present with at least one further nonionic surfactant it is present in an amount less than the amount of the at least one further noninonic which is necessarily present. Table 1 of that document illustrates that ratios of nonionic:active basis of the lauramine oxide of 1.7:1, and 8.3:1. Thus, in the reference when an amine oxide is present in the example compositions, such are present in less than 50%wt. of the cumulative total of surfactants present, when the quaternary ammonium cationic surfactant is not included in such a calculation.

PCT/GB2010/051027 discloses viscous compositions which include a ternary thickening system which necessarily includes each of (a) a benzophenone constituent, an oxyalkenylated constituent, and a fatty alkanolamide constituent. The examples of that document demonstrate compositions which necessarily include the ternary thickening system and each component thereof. Further, almost all examples (see Table 1) of that document demonstrate compositions wherein an amine oxide is included amongst further surfactants (other than the cationic compounds listed) in amounts less than 50%wt. of the cumulative weight of said surfactants, or in which a betaine surfactant is also present.

While the prior art includes certain topical compositions for use in handwash and bodywash applications, there is nonetheless a real and continuing need in the art for further improvements to such compositions. It is to these and further shortcomings in the art to which the present inventive compositions and methods for their use are directed.

In a first aspect the present invention provides a largely aqueous topical antimicrobial cleaning composition which provides an antimicrobial benefit.

In a second aspect that present inventors have found that largely aqueous topical antimicrobial cleaning compositions may be produced which include reduced amounts of an antimicrobially active quaternary ammonium chloride surfactant, wherein the composition further necessarily includes an amine oxide nonionic surfactant.

According to a third aspect, the present invention provides largely aqueous topical antimicrobial cleaning compositions may be produced which include reduced amounts of an antimicrobially active quaternary ammonium chloride surfactant, wherein the composition further necessarily includes as a sole or predominant further surfactant, an amine oxide surfactant.

A fourth aspect of the invention is a largely aqueous topical antimicrobial cleaning composition according to the first, second or third aspect of the application which is adapted for use in the application to the epidermis, e.g., hands, arms, legs, face, scalp as well as other body areas including hair.

According to a fifth aspect of the invention is provided a method for the manufacture or production of improved largely aqueous topical antimicrobial cleaning compositions as set forth herein,

According to a sixth aspect of the invention there is provided a method for providing an antimicrobial benefit to a topical surface, e.g., the epidermis or other body area or hair, which method includes the step of applying an antimicrobially effective amount of a topical germicidal composition as taught herein, preferably to reduce the incidence of undesired microorganisms present on the treated topical surface, other body surface or hair.

In a seventh aspect, the present invention provides a topical germicidal composition according to the any of the prior aspects of the invention, characterized in that the said composition is effective against one or more, preferably at least two or more, ore preferably at least 3 or more of the following microorganisms: *E. coli, S. aureus, P. aerugihosa,* and *E. hirae.*

According to a eighth aspect, there is provided a the topical germicidal compositions as described herein which may be provided in a variety of vendible product forms, e.g., viscous flowable forms, such as gels, creams or pastes as well as readily flowable forms adapted to be poured from a bottle or flask, or more flowable forms suitable to be dispensed from such a bottle, flask or other reservoir via a nozzle or a pump, e.g., a manually operable pump or an electrically driven pump as may be found in a dispensing apparatus.

These and further aspects of the invention will become more apparent from a reading of the following specification.

Broadly speaking the present inventors have found that there may be provided highly aqueous, antimicrobially effective topical compositions which comprise:
at least one first cationic surfactant or salt thereof having antimicrobial or germicidal properties or salt form thereof, preferably a quaternary ammonium cationic surfactant;
at least one different, second quaternary ammonium cationic surfactant or salt form thereof according to the structure (I) 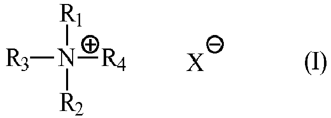
wherein:
   R₁ is a C₂₀ - C₃₆, preferably unsubstituted alkyl moiety which may optionally be branched but is preferably unbranched,
   R₂, R₃ and R₄ are independently C₁-C₃ alkyl moieties, and,
   X is a salt forming counterion which renders the second quaternary surfactant water soluble or water dispersible, and,
   an amine oxide nonionic surfactant which is the predominant further surfactant in the composition,
   wherein the compositions are in the pH range of 4.5 - 6, preferably in the pH range of 4.5 - 5.5 and which compositions are preferably low viscosity, pourable unpressurized liquids which are used as cleansing compositions for topical surfaces. In addition to the above essential constituents, the compositions may include one or more further optional constituents which may be added to provide a further technical and/or aesthetic benefit to the highly aqueous, antimicrobially effective topical compositions which further optional constituent(s) do not deleteriously affect the antimicrobial benefits provided by the system of the first cationic surfactant or salt thereof, the different, second quaternary ammonium cationic surfactant or salt thereof and the amine oxide surfactant, which is the remaining or predominantly remaining further surfactant present in the composition.

A first essential constituent of the invention is at least one cationic surfactant or salt thereof having antimicrobial or germicidal properties or salt form thereof. Examples such cationic surfactants which provide a germicidal effect to the compositions, and especially preferred such surfactants are quaternary ammonium compounds and salts thereof, which may be characterized by the general structural formula: 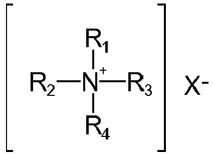 where at least one of R₁, R₂, R₃ and R₄ is a alkyl, aryl or alkylaryl substituent of from 6 to 18 carbon atoms, and the entire cation portion of the molecule has a molecular weight of at least 165. The alkyl substituents may be long-chain alkyl, long-chain alkoxyaryl, long-chain alkylaryl, halogen-substituted long-chain alkylaryl, long-chain alkylphenoxyalkyl, arylalkyl, etc. The remaining substituents on the nitrogen atoms other than the abovementioned alkyl substituents are hydrocarbons usually containing no more than 12 carbon atoms. The substituents R₁, R₂, R₃ and R₄ may be straight-chained or may be branched, but are preferably straight-chained, and may include one or more amide, ether or ester linkages. The counterion X may be any salt-forming anion which permits water solubility of the quaternary ammonium complex.

Exemplary quaternary ammonium salts within the above description include the alkyl ammonium halides such as cetyl trimethyl ammonium bromide, alkyl aryl ammonium halides such as octadecyl dimethyl benzyl ammonium bromide, N-alkyl pyridinium halides such as N-cetyl pyridinium bromide, and the like. Other suitable types of quaternary ammonium salts include those in which the molecule contains either amide, ether or ester linkages such as octyl phenoxy ethoxy ethyl dimethyl benzyl ammonium chloride, N-(laurylcocoaminoformylmethyl)-pyridinium chloride, and the like. Other very effective types of quaternary ammonium compounds which are useful as germicides include those in which the hydrophobic radical is characterized by a substituted aromatic nucleus as in the case of lauryloxyphenyltrimethyl ammonium chloride, cetylaminophenyltrimethyl ammonium methosulfate, dodecylphenyltrimethyl ammonium methosulfate, dodecylbenzyltrimethyl ammonium chloride, chlorinated dodecylbenzyltrimethyl ammonium chloride, and the like.

Preferred quaternary ammonium compounds which act as germicides and which are be found useful in the practice of the present invention include those which have the structural formula: 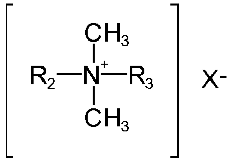 wherein R₂ and R₃ are the same or different C₈-C₁₂alkyl, or R₂ is C₁₂₋₁₆alkyl, C₈-₁₈alkylethoxy, C₈₋₁₈alkylphenolethoxy and R₃ is benzyl, and X is a halide, for example chloride, bromide or iodide, or is a methosulfate anion. The alkyl groups recited in R₂ and R₃ may be straight-chained or branched, but are preferably substantially linear.

Particularly useful quaternary germicides include compositions which include a single quaternary compound, as well as mixtures of two or more different quaternary compounds. Such useful quaternary compounds are available under the BARDAC®, BARQUAT®, HYAMINE®, LONZABAC®, and ONYXIDE® trademarks, which are more fully described in, for example, McCutcheoh's Functional Materials (Vol. 2), North American Edition, 1998, as well as the respective product literature from the suppliers identified below.

The first cationic surfactant or salt may be present in any effective amount, but generally need not be present in amounts in excess of about 2%wt. based on the total weight of the composition. The preferred first cationic surfactant (s) may be present in the compositions in amounts of from about 0.001 % by weight to up to about 2% by weight, very preferably about 0.01-1.5% by weight, more preferably in amount of between 0.05-1 % by weight, and most preferably from 0.1 - 0.5% by weight, and moreso from 0.1 - 0.4%wt. and in certain preferred embodiments in even lesser amounts. Preferred weight ranges are disclosed with reference to the examples. It is particularly advantageous that the preferred first cationic surfactant(s) is/are present in amounts of at least 0.01 parts per million (ppm), preferably in amounts of 0.05 - 1000 ppm, more preferably in amounts of from 0.1 - 500 ppm.

A second essential constituent of the inventive compositions is at least one second quaternary ammonium cationic surfactant or salt form thereof which is different from the foregoing said first cationic surfactant or salt described above. The at least one second quaternary ammonium cationic surfactant or salt form thereof conforms to the structure (I) 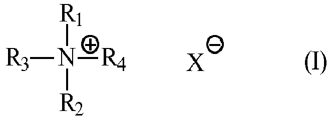 wherein:
R₁ is a C₂₀ - C₃₆, preferably unsubstituted alkyl moiety which may optionally be branched but is preferably unbranched,
R₂, R₃ and R₄ are independently C₁-C₃ alkyl moieties, and,
X is a salt forming counterion which renders the second quaternary surfactant water soluble or water dispersible.

Preferably, R₁ is a C₂₀ - C₃₆, unsubstituted and unbranched alkyl moiety and especially preferably is an unsubstituted unbranched C₂₀ - C₂₆ alkyl moiety, and concurrently R₂, R₃ and R₄ are each methyl, and X is a halide, for example chloride, bromide or iodide, or is a methosulfate anion, but especially preferably is a halide which renders the surfactant compound of the formula (I) water soluble or water dispersible. Non-limiting examples of such different, second quaternary ammonium cationic surfactants are described with reference to the following examples.

This different, second quaternary ammonium cationic surfactant or salt form need not provide an appreciable germicidal or antimicrobial benefit. In certain preferred embodiments this different, second quaternary ammonium cationic surfactant or salt form thereof provides minimal or no effective germicidal benefit. This different, second quaternary ammonium cationic surfactant or salt form thereof may be primarily provided as a cationic emulsifier.

This different, second quaternary ammonium cationic surfactant or salt form may be present in any effective amount, but generally need not be present in amounts in excess of about 10%wt. based on the total weight of the composition. The preferred first cationic surfactant (s) may be present in the compositions in amounts of from about 0.001 % by weight to up to about 10% by weight, very preferably about 0.01-8% by weight, more preferably in amount of between 0.5-6 % by weight, and most preferably from 1 - 5% by weight.

The next essential constituent of the invention is an amine oxide nonionic surfactant which is preferably the predominant, but more preferably is the sole further surfactant in the composition. Exemplary such amine oxides include:
A) Alkyl di (lower alkyl) amine oxides in which the alkyl group has about 10-20, and preferably 12-16 carbon atoms, and can be straight or branched chain, saturated or unsaturated. The lower alkyl groups include between 1 and 7 carbon atoms. Examples include lauryl dimethyl amine oxide, myristyl dimethyl amine oxide, and those in which the alkyl group is a mixture of different amine oxide, dimethyl cocoamine oxide, dimethyl (hydrogenated tallow) amine oxide, and myristyl/palmityl dimethyl amine oxide;
B) Alkyl di (hydroxy lower alkyl) amine oxides in which the alkyl group has about 10-20, and preferably 12-16 carbon atoms, and can be straight or branched chain, saturated or unsaturated. Examples are bis(2-hydroxyethyl) cocoamine oxide, bis(2-hydroxyethyl) tallowamine oxide; and bis(2-hydroxyethyl) stearylamine oxide;
C) Alkylamidopropyl di(lower alkyl) amine oxides in which the alkyl group has about 10-20, and preferably 12-16 carbon atoms, and can be straight or branched chain, saturated or unsaturated. Examples are cocoamidopropyl dimethyl amine oxide and tallowamidopropyl dimethyl amine oxide; and
D) Alkylmorpholine oxides in which the alkyl group has about 10-20, and preferably 12-16 carbon atoms, and can be straight or branched chain, saturated or unsaturated.

Preferably the amine oxide constituent is an alkyl di (lower alkyl) amine oxide as denoted above and which may be represented by the following structure: 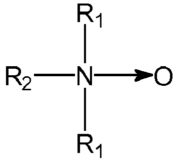 wherein each:
R₁ is a straight chained C₁-C₄ alkyl group, preferably both R₁ are methyl groups; and,
R₂ is a straight chained C₈-C₁₈ alkyl group, preferably is C₁₀-C₁₄ alkyl group, most preferably is a C₁₂ alkyl group.
Each of the alkyl groups may be linear or branched, but most preferably are linear. Most preferably the amine oxide constituent is lauryl dimethyl amine oxide. Technical grade mixtures of two or more amine oxides may be used, wherein amine oxides of varying chains of the R₂ group are present. Preferably, the amine oxides used in the present invention include R₂ groups which comprise at least 50%wt., preferably at least 60%wt. of C₁₂ alkyl groups and at least 25%wt. of C₁₄ alkyl groups, with not more than 15%wt. of C₁₆, C₁₈ or higher alkyl groups as the R₂ group.

While the nonionic amine oxide surfactants are necessarily present, and while they may be included in any effective or desired amount, advantageously they comprise up to 15%wt, but preferably less of the composition of which they form a part. Advantageously the amine oxide nonionic surfactants comprise between 0.001 - 15%wt, more preferably 0.1 - 10%wt., still more preferably 1 - 8%wt. of the largely aqueous topical antimicrobial cleaning compositions of which they form a part. Particularly preferred nonionic amine oxide surfactants as well as particularly preferred amounts of such nonionic amine oxide surfactants are disclosed with reference to one or more of the examples.

It is also a characteristic feature of the present invention that the amine oxide surfactant provides at least 50%wt. of the cumulative total of surfactants present, when the quaternary ammonium cationic surfactant is not included in such a calculation.

The final essential constituent of the largely aqueous topical antimicrobial cleaning composition is water and it provides at least 70%wt. to 100%wt. of the compositions of the invention. The water may be tap water, but is preferably distilled and is most preferably deionized water or "soft" water. If the water is tap water, it is preferably substantially free of any undesirable impurities such as organics or inorganics, especially minerals salts which are present in hard water which may thus undesirably interfere with the operation of the constituents present in the topical germicidal compositions according to the present invention. Advantageously water is included in the compositions in amounts of at least 70%wt, and preferably (and in order of increasing preference) at least 71%wt., 72%wt., 73%wt., 74%wt., 75%wt., 76%wt., 77%wt., 78%wt., 79%wt., 80%wt., 81%wt., 82%wt., 83%wt., 84%wt., 85%wt., 86%wt., 87%wt., 88%wt., 89%wt., 90%wt., 91%wt., 92%wt., 93%wt., 94%wt., and 95%wt., based on the total weight of the largely aqueous topical antimicrobial cleaning composition of which water forms a part.

The present inventors have surprisingly found that when an amine oxide nonionic surfactant is included as the predominant, and in some cases, the sole further surfactant present in addition to the at least one first cationic surfactant having antimicrobial or germicidal properties or salt form thereof and with the at least one different, second quaternary ammonium cationic surfactant or salt form thereof, the resultant largely aqueous topical antimicrobial cleaning compositions provide a high degree of antimicrobial efficacy even when the amounts of the at least one first cationic surfactant and the at least one different, second quaternary ammonium cationic surfactant are present in reduced amounts as compared to similar compositions wherein the amine oxide nonionic surfactant is neither the predominant, nor the sole further surfactant present in those other similar compositions. This result is surprising and unexpected, and permits for the formulation of largely aqueous topical antimicrobial cleaning composition with reduced amounts of the at least one first cationic surfactant having antimicrobial or germicidal properties or salt form thereof and the at least one different, second quaternary ammonium cationic surfactant or salt form thereof, which possibility is advantageous from both an economic standpoint in that lesser amounts of the at least one first cationic surfactant having antimicrobial or germicidal properties or salt form thereof and with the at least one different, second quaternary ammonium cationic surfactant or salt form thereof are required in order to provide a desired degree of antimicrobial effect, as well as from a technical perspective as the at least one first cationic surfactant having antimicrobial or germicidal properties or salt form thereof may in certain formulations exhibit a small degree of dermal or ocular irritancy, which is reduced or eliminated with reduced amounts of the foregoing cationic compounds while at the same time, not diminishing the desired antimicrobial or germicidal properties desired.

While other surfactants, e.g., anionic, nonionic, cationic, amphoteric or zwitterionic surfactants. may optionally be present, in addition to the amine oxide nonionic surfactant, this amine oxide nonionic surfactant is most desirably the "predominant" or sole surfactant present other than the at least one first cationic surfactant having antimicrobial or germicidal properties or salt form thereof and with the at least one different, second quaternary ammonium cationic surfactant or salt form thereof which are also necessarily present. By "predominant" is to be understood that the nonionic surfactant comprises at least 50%wt. but preferably (in order of increasing preference, in weight percentages) at least 50.5%, 51%, 52%, 54%, 56%, 58%, 60%, 62%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5% of the total weight of all surfactants present in the largely aqueous topical antimicrobial cleaning composition other than the amounts of the at least one first cationic surfactant having antimicrobial or germicidal properties or salt form thereof and with the at least one different, second quaternary ammonium cationic surfactant or salt form thereof which are not taken into consideration in such an assessment. In certain particularly preferred embodiments, the amine oxide nonionic surfactant is most desirably the sole surfactant present, other than the at least one first cationic surfactant having antimicrobial or germicidal properties or salt form thereof and with the at least one different, second quaternary ammonium cationic surfactant or salt form.

The inventive compositions exhibit a pH in the range of from about 4.5 to about 6, preferably a pH in the range of from about 4.5 to about 5.5., and most preferably between about 4.5 and about 5. Particularly preferred pH ranges are disclosed with reference to one or more of the examples. When necessary a pH adjusting agent or constituent may be used to provide and/or maintain the pH of a composition.

In a further aspect of the invention there is provided a germicidal and surfactant "system" which comprises (or consists essentially of, or consists of) each of the foregoing essential constituents in the amounts indicated, and especially in the preferred amounts indicated, which said germicidal and surfactant "system" is aqueous and has pH in the ranges described herein. This system is sufficient to provide an excellent germicidal benefit when incorporated into a topical compositions for use in handwash and bodywash applications, and especially when the said system is present in a foaming topical antimicrobial cleansing compositions which may optionally include one or further constituents if so desired. In certain embodiments however, the constituents of the foregoing system are the sole constituents present in the inventive compositions which provide a germicidal and/or foaming effect.

Optionally, the compositions of the invention may include one or more further constituents which may be used to improve one or more aesthetic and/or technical characteristics of the composition of which they form a part. Typically they are included in only small amounts, and usually the total amount of any such optional constituents does not exceed 25%wt. of the inventive compositions of which they form a part. In certain preferred embodiments of the invention, one or more of the following recited optional constituents may be considered as essential constituents according to a particular preferred embodiment. Such optional constituents include additives and adjuvants which are conventional in the cosmetic, pharmaceutical or dermatological field, such as hydrophilic or lipophilic gelling agents, further surfactants other than those listed as essential constituents, emulsifiers, particulates, fillers, emollients, skin conditioning agents, preservatives, antioxidants, solvents especially organic solvents, pH adjusting agents, pH buffers, chelating agents, fragrances or other materials which provide an aromatherapy benefit, fillers, preservatives, dyestuffs or colorants, opacifiers, as well as light stabilizers including UV absorbers.

The compositions may include one or more further anionic, nonionic, cationic, amphoteric or zwitterionic surfactants. By way of non-limiting examples of such nonionic surfactants include ethoxylated fatty alcohols, polyethoxylated fatty alcohols, glycerol mono-fatty acid esters, fatty acid esters of polyethylene glycol, polyethoxylated sorbitan fatty acid esters, and alkylglycosides. Such surfactants may be useful as emulsifier constituents. Particularly preferred optional surfactants are further described with reference to one or more of the examples. Such surfactants may be present in any effective amount, and when included, advantageously are present in amounts of from about 0.01%wt. to about 7.5%wt., preferably from about 0.25%wt. to about 2.5%wt., based on the total weight of the composition of which they form a part.

One preferred nonionic surfactant which is advantageously included in the inventive compositions (and in certain further aspects of the invention, is a further essential constituent) are one or more nonionic surfactants based on alkanolamide compounds. Exemplary useful alkanolamide compounds include one or more monoethanol amides, and diethanol amides of fatty acids having an acyl moiety which contains from about 8 to about 18 carbon atoms, and which may be represented in accordance with the formula:

R₁-CO-N(H)ₘ₋₁(R₂OH)₃₋ₘ

where R₁ represents a saturated or unsaturated aliphatic hydrocarbon radical of from about 7 to 21 carbon atoms, but preferably from about 11 to 17 carbon atoms; R₂ represents a -CH₂- or -CH₂CH₂-, and m is an integer from 1 to 3, but is preferably 1. Preferably, R₁ is a saturated or unsaturated aliphatic hydrocarbon radical comprising from about 11 to 17 carbon atoms, and m is 1. Specific examples of such compounds include monoethanol amine coconut fatty acid amide and diethanol amine dodecyl fatty acid amide. An exemplary useful and particularly preferred fatty acid amides include cocomonoethanol amide or cocodiethanolamide, which are presently commercially available as MONAMID CMA or MONAMID MDNA (ex. Mona Industries, Paterson NJ). Further exemplary useful alkanolamides which provide such functions include *inter alia:* cocamide MEA, cocamide DEA, soyamide DEA, lauramide DEA, oleamide MIPA, stearamide MEA, myristamide MEA, lauramide MEA, capramide DEA, ricinoleamide DEA, myristamide DEA, stearamide DEA, oleylamide DEA, tallowamide DEA, lauramide MIPA, tallowamide MEA, isostearamide DEA, isostearamide MEA, and mixtures thereof. Further useful alkanolamide surfactant compounds include alkanolamides, particularly fatty monoalkanolamides and fatty dialkanolamides, including one or more of those marketed under the Ninol® tradename, e.g., Ninol® 55-LL. Further exemplary alkanolamide surfactant compounds include monoethanol amides and diethanol amides include those marketed under the trade names Alakamide® and Cyclomide® by Rhone-Poulenc Co., (Cranbury, NJ) e.g., Cyclomide® CDD-518 described to be a nonionic surfactant based on coconut diethanolamide; Cyclomide® C212 described to be a nonionic surfactant based on coconut monoethanolamide; Cyclomide® DC212/SE described to be a nonionic surfactant based on 1:1 fatty acid diethanolamide; Cyclomide® DIN 100 described to be a nonionic surfactant based on lauric/linoleic diethanolamide; Cyclomide® DIN-295/S described to be a nonionic surfactant based on 1:1 linoleic diethanolamide; Cyclomide® DL203 described to be a nonionic surfactant based on 2:1 lauric diethanolamide.

Advantageously when one or more further optional surfactants are included, the total amount of such further optional surfactants are present in amount of up to but not including 50%wt. based on the total weight of all surfactants present in the compositions, not taking into consideration the first and second essential cationic surfactants which are essential to the inventive compositions. Furthermore, in certain particularly preferred embodiments certain surfactants are necessarily excluded from the compositions, e.g., in certain preferred embodiments the inventive compositions exclude anionic surfactants, as such may interfere with the essential cationic compounds which are necessarily present. Thus, in preferred embodiments the compositions are free of anionic surfactants and anionic soaps and are desirably also free of amphoteric surfactants. Yet, the compositions exhibit a satisfactory degree of foaming and exhibit excellent antimicrobial efficacy to treated surfaces, particularly a body surface, e.g., epidermis, skin, scalp and hair. The topical antimicrobial compositions are excellently suited for animal use, including human use.

The topical antimicrobial compositions thus most desirably exclude anionic surfactants and/or salt forms thereof. Examples of classes of anionic surfactants excluded are sulfates, sulfonates, phosphates, phosphonates and carbonates. Anionic soap surfactants are also most desirably excluded from the inventive compositions. Advantageously, the topical antimicrobial compositions also exclude amphoteric surfactants. Examples of classes of amphoteric surfactants excluded are derivatives of aliphatic secondary and tertiary amines wherein at least one of the aliphatic substituents contains an anionic water-solubilizing group, e.g., a carboxy, sulfonate, or a sulfate group. Amphoteric surfactants which are especially desirably excluded include betaines

While one or more conventional thickeners based on thickeners, gums, clays or polymers may be present as optional constituents, in particularly preferred embodiments, such one or more of the thickeners are expressly excluded from the compositions of the present invention. Benzophenones are also preferably expressly excluded from the inventive compositions. In certain particularly preferred embodiments the inventive compositions have a viscosity which is not more than 15%, preferably not more than 10%, and particularly preferably not more than 5% of deionized water when tested at like conditions. In other particularly preferred embodiments the inventive compositions exhibit a viscosity of between about 1 - 200 cPs, preferably between 1 - 150 cPs, more preferably between 1 - 100 cPs when measured using a Brookfield viscometer, utilizing spindle 1 at 60 rpm, measured in an open beaker at normal atmospheric pressure and at room temperature (20° - 22°C).

The compositions of the invention may optionally comprise one or more emollients which provide softness to the largely aqueous topical antimicrobial cleaning compositions. Non-limiting examples of useful emollients include those, for example, compounds based on Guerbet alcohols based on fatty alcohols containing 6 to 18 and preferably 8 to 10 carbon atoms and other additional esters, such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of C₁₈₋₃₈ alkyl-hydroxycarboxylic acids with linear or branched C₆₋₂₂ fatty alcohols, more especially dioctyl malate, esters of linear and/or branched fatty acids with polyhydric alcohols (for example propylene glycol, dimer diol or trimer triol), triglycerides based on C₆₋₁₀ fatty acids, liquid mono-, di- and triglyceride mixtures based on C₆₋₁₈ fatty acids, esters of C₆₋₂₂ fatty alcohols and/or Guerbet alcohols with aromatic carboxylic acids, more particularly benzoic acid, esters of C₂₋₁₂ dicarboxylic acids with polyols containing 2 to 10 carbon atoms and 2 to 6 hydroxyl groups, vegetable oils, branched primary alcohols, substituted cyclohexanes, linear and branched C₆₋₂₂ fatty alcohol carbonates such as, for example, dicaprylyl carbonate (commercially available as Cetiol® CC), Guerbet carbonates based on fatty alcohols containing 6 to 18 and preferably 8 to 10 carbon atoms, esters of benzoic acid with linear and/or branched C₆₋₂₂ alcohols (for example, a product commercially available as Finsolv® TN), linear or branched, symmetrical or nonsymmetrical dialkyl ethers containing 6 to 22 carbon atoms per alkyl group such as, for example, dicaprylyl ether (commercially available as Cetiol® OE), ring opening products of epoxidized fatty acid esters with polyols and hydrocarbons or mixtures thereof (commercially available as Cetiol® DD), propylheptyl caprylate (commercially available as Cetiol® SenSoft) as well as the compounds disclosed in published US Patent application 2009/0182046 the contents of which are herein incorporated by reference.

The largely aqueous topical antimicrobial cleaning compositions may include a cosmetic particulate, which may be any particulate material which is a solid at room temperature (approx. 20°C) temperature and atmospheric pressure, which does not deleteriously react chemically with balance of the constituents of the inventive composition. Advantageously the cosmetic particulate is insoluble in balance of the constituents of the largely aqueous topical antimicrobial cleaning compositions, particularly when the compositions are brought to a temperature above room temperature and especially to a temperature of at least 50°C and preferably at least 60°C for at least 24 hours, preferably for at least 48 hours. Desirably the cosmetic particulate constituent exhibits a melting temperatures of at least 70°C, preferably at least 100°C, more preferably at least 120°C, and most preferably at least 130°C. The cosmetic particulate composition may be absorbent or non-absorbent with respect to one or more of the remaining constituents of the inventive compositions of which they form a part..

Advantageously the cosmetic particulate constituent may be mineral or organic, lamellar, spherical, viz., beads, or oblong. They may have a generally regular geometry, such as in the case of spheres or rods, or they may have an irregular geometry such as crushed particulate materials. Exemplary materials useful for the cosmetic particulate constituent include: inorganic particulate particles formed from talc, mica, silica, kaolin, boron nitride, carbonates such as precipitated calcium carbonate, magnesium carbonate and magnesium hydrocarbonate, hydroxyapatite, hollow silica microspheres, glass microcapsules, and ceramic microcapsules, inorganic pigments and mixtures thereof. Exemplary materials useful for the cosmetic particulate constituent include: organic particulate particles formed from polyamide powders, such as polyamides (Nylons), polyethylenes, polypropylenes, polyesters, acrylic polymers such as polymethyl methacrylate, polytetrafluoroethylene (Teflons.), as well as crystalline and microcrystalline waxes derived from plants, mineral oils or petroleum, hollow polymer microspheres such as those formed from polyvinylidene chloride/acrylonitrile, starches, alginates, organic dyestuffs or pigments, and mixtures thereof. Mixtures of two or more cosmetic particles may be used to provide the cosmetic particulate constituent. Preferred as the cosmetic particulate constituent are materials which provide an exfoliating benefit.

Preferably, these cosmetic particulates have an apparent diameter in the range of from about 100 to about 1000 µm, preferably from about 100 to about 600 µm and most preferably from about from about 250 to about 600µm. An apparent diameter corresponds to the diameter of the circle in which the elementary particle is inscribed along its smallest dimension (thickness for lamellae).

A preferred class of cosmetic particulate materials are based on synthetically occurring or synthetic waxes inclusive of microcrystalline waxes. Exemplary useful waxes include any of those which are generally useful used in cosmetics and dermatology. Exemplary waxes of natural origin, include for instance beeswax, carnauba wax, candelilla wax, ouricoury wax, Japan wax, cork fibre wax or sugar cane wax, paraffin wax, lignite wax, microcrystalline waxes, lanolin wax, montan wax, ozokerites, hydrogenated oils, for instance hydrogenated jojoba oil. Exemplary waxes of synthetic origin include for instance polyethylene waxes derived from the polymerization of ethylene, waxes obtained by Fischer-Tropsch synthesis, esters of fatty acids and of glycerides that are solid at 50°C. preferably at 60°C or higher temperatures, and silicone waxes, for instance alkyl, alkoxy, and/or esters of poly(di)methylsiloxane that are solid at 50°C. preferably at 60°C or higher temperatures. These waxes may be formed particulates, e.g., beads or spheres according to conventional methods.

The cosmetic particulate constituent of the invention may be provided in any effective amount, but desirably is present in amount which are aethetically pleasing to the user of the composition. The cosmetic particulate constituent is made of individual cosmetic particulate materials which may be of a uniform chemical or physical composition, and/or of a uniform size or dimension and/or of a uniform color but this is not a necessity and mixtures or different individual cosmetic particulate materials which may be differentiated on the basis of chemical and/or physical composition, and/or size or dimension and/or color may be provided as the cosmetic particulate constituent of the invention. If included in the compositions of the invention, the cosmetic particulate constituent of the invention may be provided in any effective amount, advantageously from at least 0.01%wt., preferably at least 0.05%wt, and most preferably at least 0.1%wt of the largely aqueous topical antimicrobial cleaning composition. Similarly advantageously the cosmetic particulate constituent is present in not more than 10%wt., preferably not more than 5%wt, and yet more preferably not more than 2%wt, and most preferably not more than 2%wt of the largely aqueous topical antimicrobial cleaning composition of which it forms a part.

The largely aqueous topical antimicrobial cleaning compositions may include one or more fillers in the form of powders. By way of non-limiting examples these powders include chalk, talc, kaolin, starch, smectite clays, chemically modified magnesium aluminum silicate, organically modified montmorillonite clay, hydrated aluminum silicate, fumed silica, aluminum starch octenyl succinate and mixtures thereof. When present in a composition, the one or more fillers may be present in amounts of up to about 5%wt., preferably are present in amounts of from about 0.001%wt. to about 5%wt. based on the total weight of the topical composition of which it forms a part.

The compositions of the invention may optionally include one or more polysiloxanes which are commonly used and often interchangeably referred to as silicone emulsifiers. Such silicone emulsifiers include polydiorganosiloxanepolyoxyalkylene copolymers containing at least one polydiorganosiloxane segment and at least one polyoxyalkylene segment. The polyoxyalkylene segments may be bonded to the polydiorganosiloxane segments with silicon-oxygen-carbon bonds and/or with silicon-carbon bonds. The polydiorganosiloxane segments of consist essentially of siloxane units which are interlinked by Si-O-Si linkages and which have the formula:

R_{b}SiO_{(4{b})/2}

The value of b may range from 0 to 3 for said siloxane units with the provision that there is an average of approximately 2, i.e. from 1.9 to 2.1 R radicals for every silicon in the copolymer. Suitable siloxane units thus include R₃SiO_{1/2}, R₂SiO_{2/2}, RSiO_{3/2}, and SiO_{4/2} siloxane units taken in such molar amounts so that b has an average value of approximately 2 in the copolymer. Said siloxane units may be arranged in linear, cyclic and/or branched fashion. The R radicals may be any radical selected from the group consisting of methyl, ethyl, vinyl, phenyl, and a divalent radical bonding a polyoxyalkylene segment to the polydiorganosiloxane segment. At least 95 percent of all R radicals are methyl radicals; preferably there is at least one methyl radical bonded to each silicon atom in (d). Divalent R radicals preferably contain no more than 6 carbon atoms. Examples of divalent R radicals include --O--, --CₘH₂ₘO--, --CₘH₂ₘ-- and --CₘH₂ₘCO₂-- where m is an integer greater than zero. Illustrative of the siloxane units that make up the polydiorganosiloxane segments are the following, where Me denotes methyl and Q denotes said divalent R radical and bonded polyoxyalkylene segment: R₃SiO_{1/2} units such as Me₃SiO_{1/2}, Me₂(CH₂=CH)SiO_{1/2}, Me₂(C₆ H₅)SiO_{1/2}, Me(C₆H₅)(CH₂=CH)SiO_{1/2}, Me₂(CH₃CH₂)SiO_{1/2}, Me₂QSiO_{1/2}, MeQ₂ SiO_{1/2}, Q₃SiO_{1/2}, Q₂(CH₃CH₂)SiO_{1/2}, and Me(C₆H₅)(Q)SiO_{1/2}; R₂SiO_{2/2} units such as Me₂SiO_{2/2}, Me(C₆H₅)SiO_{2/2}, Me(CH₂=CH)SiO_{2/2}, (C₆H₅)₂SiO_{2/2}, MeQSiO_{2/2} and Q(C₆H₅)SiO_{2/2} ; RSiO_{3/2} units such as MeSiO_{3/2}, C₆H₅SiO_{3/2}, CH₂=CHSiO_{3/2} CH₃CH₂SiO_{3/2} and QSiO_{3/2}; and SiO_{4/2} units.

Volatile linear silicones including polydimethylsiloxane and dimethicones may also be present as silicone emulsifiers in compositions according to the invention.

Also useful as silicone emulsifiers in the inventive compositions are one or more compounds which may be represented by the structure: 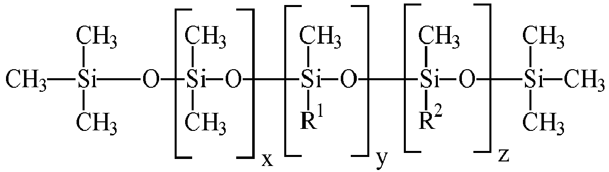 wherein
R¹ represents a C₁-C₃₀ straight chained, branched or cyclic alkyl group,
R₂ represents a moiety selected from:

-(CH₂)ₙ-O-(CH₂CHR³O)ₘ-H

and

-(CH₂)ₙ-O-(CH₂CHR³O)ₘ-(CH₂CHR⁴O)ₚ-H

in which n represents an integer from about 3 to about 10, R3 and R4 are sleeted from hydrogen and C1-C6 straight chain, or branched chain alkyl groups with the proviso that R³ and R⁴ are not simultaneously the same, each of m, p, x and y are independently selected from integers of zero or greater, such that the molecule has a molecular weight of between about 200 to about 20,000,000 and wherein both m and p are not both simultaneously zero, and z is selected from integers of 1 or greater.

If included in the compositions of the invention, the silicone emulsifiers may be provided in any effective amount, advantageously from at least 0.01%wt., preferably at least 0.05%wt, of the composition. Advantageously the silicone emulsifiers, when present, are present in amounts of not more than 5%wt, and yet more preferably not more than 2%wt, and most preferably not more than 2%wt of the composition of which it forms a part.

The largely aqueous topical antimicrobial cleaning compositions may include one or more powders or pulvurent materials. These powders include mica, chalk, talc, Fullers earth, kaolin, starch, silica, silicates, hydrated aluminum silicate, fumed silica, aluminum starch octenyl succinate as well as comminuted or particulate polymers such as particles of polyamides (Nylons), polyalkyleneterephtalates (PET, PBT), polyolefins (PE) or fluoropolymers (polytetrafluoroethylene) as well as mixtures of two or more thereof. The inclusion of one or more powders in the inventive compositions may provide an improved tactile benefit and/or may act to absorb a part of one or more of the hydrophobic constituents present in the composition and/or may provide an opacifying effect to the compositions. Preferred powders are those based on inorganic materials, e.g., silica, silicates and talc. Such are typically provided to the largely aqueous topical antimicrobial cleaning compositions as finely divided particles. While such powders may be included in any effective amount, when present they are advantageously included in amounts of between about 0.01%wt. to about 5%wt., preferably between about 0.25%wt. to about 2%wt., based on the total weight of the largely aqueous topical antimicrobial cleaning composition of which they form a part.

The largely aqueous topical antimicrobial cleaning compositions may include which comprise one or more paraffinic hydrocarbons and/or preparations containing the same. Such paraffinic hydrocarbons may include one or both of linear and/or branched paraffinic hydrocarbons. Mixtures of branched hydrocarbons especially as isoparaffins form a further particularly preferred form of a useful hydrocarbon solvent of the invention. Particularly useful technical grade mixtures of isoparaffins include mixtures of isoparaffinic organic solvents having a relatively narrow boiling range. Examples of these commercially available isoparaffinic organic solvents include those consisting substantially of linear isoparaffins, e.g., those commercially available as Norpar® solvents (ex. ExxonMobil Corp.) as well as those containing branched isoparaffins, e.g., those commercially available as Isopar® solvents (ex. ExxonMobil Corp.) Further, other preparations which include a significant proportions of one or more isoparaffins may also be utilized. Such include, for example, SiClone® SR-5, (ex. Presperse LLC, Somerset, NJ (USA)) which is described to be a technical mixture of C₁₃-C₁₆ isoparaffins, C₁₂-C₁₄ isoparaffins, with a C₁₃-C₁₅ alkane constituent, which technical mixture is marketed as a substitute for cyclomethicone in cosmetic formulations, yet is 100% silicone-free.

While such paraffinic hydrocarbons and/or preparations containing the same may be included in any effective amount, when present they are advantageously included in amounts of between about 0.01%wt. to about 5%wt., preferably between about 0.1%wt. to about 2%wt., based on the total weight of the largely aqueous topical antimicrobial cleaning composition of which they form a part.

A further optional constituent which may be included in the inventive compositions are oxyalkylenated compounds. Such oxyalkylenated compounds are not considered to be surfactants in the present inventive compositions, or are considered in the calculation of the percentages of or weight ratios of the amine oxide surfactants to the other non-cationic surfactants which may be present in the compositions. Exemplary oxyalkylenated compound(s) which may be used in the composition of the invention may comprise ethylene oxide groups (oxyethylenated compounds), propylene oxide groups (oxypropylenated compounds) or both (oxyethylenated/oxypropylenated compounds).

Suitable oxyalkylenated compounds include, in particular, polyethylene glycols, polyethylene glycol esters and/or polypropylene glycol esters, polyethylene glycol ethers and/or polypropylene glycol ethers, alkoxylated aryl derivatives and in particular ethoxylated aryl polyol derivatives, oxyalkylenated and in particular oxyethylenated triesters of glycerol and of fatty acids, ethoxyethylenated urethane derivatives modified with alkyl chains, and mixtures thereof.

Exemplary polyethylene glycols which may be used in the composition of the invention include ethylene oxide polycondensates having a number of ethylene oxide (EO) units of greater than 100. The ethylene oxide number may range, for example, from 100 to 50 000. but preferably from 100 to 500, more preferably from 100 - 200. Suitable examples of polyethylene glycols include polyethylene glycol comprising 7 000 EO (CTFA name: PEG-7M), polyethylene glycol comprising 75 EO (CTFA name: PEG-75), polyethylene glycol comprising 20,000 EO (CTFA name: PEG-20M) and polyethylene glycol comprising 150 EO (CTFA name: PEG-150).

Also useful as oxyalkylenated compounds are polyethylene glycol esters and/or polypropylene glycol esters which are condensates of polyethylene glycol and/or polypropylene glycol with one or more fatty acids. These compounds have the formula:

RCOO-(EO)ₘ-(PO)ₙ-R'

in which m has a value of 0 to 300, n has a value of 0 to 300 the sum of m and n is 6 or greater, and R and R' represent, independently of each other, hydrogen or a saturated or unsaturated, linear or branched, hydroxylated or non-hydroxylated alkyl chain containing from 1 to 30 carbon atoms and preferably from 12 to 22 carbon atoms, or an aryl chain, with the proviso that R and R' are not simultaneously hydrogen.

Suitable, non-limiting examples of polyethylene glycol acid esters and/or polypropylene glycol acid esters include polyethylene glycol distearate (150 EO), PEG-150 dibehenate, polyethylene glycol palmitostearate (120 EO), the copolymer of polyethylene glycol ligand (30 EO) and of 12-hydroxystearic acid and polyethylene glycol stearate (40 EO), as well as such compounds based on polyoxyethylene/polyoxypropylene copolymers.

Further useful compounds include polyethylene glycol ethers and/or polypropylene glycol ethers are condensates of polyethylene glycol and/or polypropylene glycol with one or more fatty alcohols. These are compounds of the formula:

R-(EO)ₘ-(PO)ₙ-R'

in which m has a value of 0 to 300, n has a value of 0 to 300 the sum of m and n is 6 or greater, and R and R' represent, independently of each other, hydrogen or a saturated or unsaturated, linear or branched, hydroxylated or non-hydroxylated alkyl chain containing from 1 to 30 carbon atoms and preferably from 12 to 22 carbon atoms, or an aryl chain, with the proviso that R and R' are not simultaneously hydrogen.

Suitable, albeit non-limiting examples of such polyethylene glycol ethers include, in oxyethylenated (30 EO) cetyl alcohol, oxyethylenated (15 EO) oleyl alcohol, oxyethylenated (50 EO) oleyl alcohol, and oxyethylenated (21 EO) stearyl alcohol.

Suitable non-limiting examples of polyethylene glycol/polypropylene glycol ethers include oxyethylenated (5 EO) oxypropylenated (5 PO) lauryl alcohol, and oxyethylenated (25 EO) oxypropylenated (25 PO) lauryl alcohol.

Further suitable oxyalkylenated compounds include the ethoxylated alkyl or aryl derivatives of polyol include, for example, oxyethylenated derivatives of fatty acid esters or of fatty alcohol ethers and of a polyol such as glycerol, sorbitol, glucose or pentaerythritol. Non-limiting examples of such compounds include, oxyethylenated (78 EO) glyceryl cocoate, oxyethylenated (120 EO) methylglucose dioleate, and oxyethylenated (150 EO) pentaerythrityl tetrastearate.

Further suitable compounds include oxyalkylenated glyceryl triesters of fatty acids, for example, oxyethylenated (6 EO) caprylic/capric acid glycerides, and oxyethylenated (50 EO) olive oil.

Other oxyalkylenated compounds falling within the above descriptions, although not specifically disclosed herein but known to the art may also be used. Preferred oxyalkylenated compounds are disclosed with reference to one of more of the following Examples. The oxyalkylenated compounds may be present as single compounds or as mixtures of two or more oxyalkylenated compounds. In preferred embodiments the oxyalkylenated compounds exhibit a molecular weight of at least 200, and may have molecular weights as high as the range of 1000 - 10,000 or may be even higher. While such oxyalkylenated compounds, when present, may be included in any effective amount, they are advantageously included in amounts of between about 0.01%wt. to about 5%wt., preferably between about 0.25%wt. to about 2.5%wt., based on the total weight of the composition of which they form a part.

The largely aqueous topical antimicrobial compositions may include minor amount, generally not more than 5%wt., but preferably not more than (in order of increasing preference: 4.75%, 4.5%, 4.25%, 4%, 3.75%, 3.5%, 3.25%, 3%, 2.9%, 2.8%, 2.7%, 2.6%, 2.5%, 2.4%, 2.3%, 2.2%, 2.1%, 1.9%, 1.8%, 1.75%, 1.7%, 1.6%, 1.5%, 1.4%, 1.3%, 1.25%, 1.2%, 1.1%, 1%, 0.9%, 0.8%, 0.75%, 0.7%, 0.6%, 0.5%, 0.4%, 0.3%, 0.25%, 0.2%, 0.1%, 0.05% or 0% by weight of one or more organic solvents. By way of non-limiting example exemplary useful organic solvents which may be included in the inventive compositions include those which are at least partially water-miscible such as alcohols (e.g., low molecular weight alcohols, such as, for example, ethanol, propanol, isopropanol, and the like), glycols (such as, for example, ethylene glycol, propylene glycol, hexylene glycol, and the like), water-miscible ethers (e.g. diethylene glycol diethylether, diethylene glycol dimethylether, propylene glycol dimethylether), water-miscible glycol ether (e.g. propylene glycol monomethylether, propylene glycol mono ethylether, propylene glycol monopropylether, propylene glycol monobutylether, ethylene glycol monobutylether, dipropylene glycol monomethylether, diethyleneglycol monobutylether), lower esters of monoalkylethers of ethylene glycol or propylene glycol (e.g. propylene glycol monomethyl ether acetate), and mixtures thereof.

It is to be noted that certain of the foregoing organic solvents, e.g, certain glycols or glycol ethers may also concurrently function as a humectant, or as a carrier for a fragrance compound or material.

The largely aqueous topical antimicrobial compositions may include one or more preservatives. Exemplary useful preservatives include compositions which comprise parabens, including methyl parabens and ethyl parabens, glutaraldehyde, formaldehyde, 2-bromo-2-nitropropoane-1,3-diol, 5-chloro-2-methyl-4-isothiazolin-3-one, 2-methyl-4-isothiazoline-3-one, and mixtures thereof. Further suitable preservatives include thos marketed as: KATHON CG/ICP, KATHON CG/ICP II (ex. Rohm and Haas Inc.), PROXEL (ex. Zeneca), SUTTOCIDE A (ex. Sutton Laboratories) and TEXTAMER 38AD (ex. Calgon Corp.) When present the preservative is included in any amount found to be effective in retarding or inhibiting the grown of undesired microorganisms in the largely aqueous topical antimicrobial cleaning compositions, particularly during storage for several months at room temperature. The preservative composition is advantageously present in amounts of up to about 1.5%wt., preferably from about 0.00001%wt. to about 0.5%wt., most from about 0.0001 %wt. to 0.25%wt. based on the total weight of the topical composition of which it forms a part. Usually however, in light of the high alcohol content such preservatives are not required and are advantageously omitted.

The largely aqueous topical antimicrobial cleaning compositions may include a fragrance constituent, which may be based on natural and synthetic fragrances and most commonly are mixtures or blends of a plurality of such fragrances, optionally in conjunction with a carrier such as an organic solvent or a mixture of organic solvents in which the fragrances are dissolved, suspended or dispersed. When present in a composition, the fragrance constituent may be present in any effective amount such that it can be discerned by a consumer of the largely aqueous topical antimicrobial cleaning composition, however is advantageously present in amounts of up to about 5%wt., preferably from about 0.00001%wt. to about 1.5%wt., most preferably from about 0.0001%wt. to 0.25%wt. based on the total weight of the composition of which it forms a part.

The inventive largely aqueous topical antimicrobial cleaning compositions may include one or more colorants, e.g, dyes or pigments which are known to the art be useful in cosmetic or topical compositions which may be used to impart a desired color or tint to the inventive compositions. Exemplary colorants include pigments, inter alia, inorganic red pigments, such as iron oxide, iron hydroxide and iron titanate; inorganic brown pigments, such as gamma-iron oxide; inorganic yellow pigments, such as iron oxide yellow and loess; inorganic black pigments, such as iron oxide black and carbon black; inorganic violet pigments, such as manganese violet and cobalt violet; inorganic green pigments, such as chromium hydroxide, chromium oxide, cobalt oxide and cobalt titanate; inorganic blue pigments, such as Prussian blue and ultramarine blue; lakes of tar pigments; lakes of natural dyes; and synthetic resin powder complexes of the inorganic pigments as recited above. Advantageously one or more colorants may be added in amounts of about 0.001 %wt. to about 0.1% by weight, based on the total weight of the composition of which the colorant(s) forms a part.

The largely aqueous topical antimicrobial cleaning compositions of the invention may one or more essential oils which are selected to provide a so-called "aromatherapy benefit" or "holistic benefit" to the user. Essential oils are complex mixtures of different organic molecules, such as terpenes, alcohols, esters, aldehydes, ketones and phenols. Such essential oils are frequently extracted from naturally occurring botanical sources such as flowers, stems, leaves, roots and barks of aromatic plants. While essential oils may be used singly, it is also common to utilize blends of essential oils in order to provide a conjunctive aroma benefit, aromatherapy benefit, holistic benefit and possibly a therapeutic benefit as well.

Preferred essential oils providing an aromatherapy benefit for use in the largely aqueous topical antimicrobial cleaning compositions of the present invention include one or more selected from chamomile oil, lavendin oil, lavender oil, grapefruit oil, lemon oil, line oil, mandarin orange oil, orange flower oil and orange oil. Chamomile oil may be used to promote both a fresh, clean and attractive scent and possibly provide a stress-relaxing benefit to the user of the topical composition. Lavender oil, and lavendin, may be used to promote both a fresh and attractive scent and possibly also provide a stress-relaxing benefit to the user of the topical composition. One or more of grapefruit oil, lemon oil, line oil, mandarin orange oil, orange flower oil and orange oil provide a clean citrus scent and may possibly impart a perceived therapeutic benefit as well when used.

When used in the present invention, these one or more essential oils providing an aromatherapy benefit or holistic benefit are present in an amount about 0.00001 wt. % to about 1 wt. %, preferably from about 0.00005 wt. % to about 0.75 wt. %, and more preferably from about 0.0001 wt. % to about 0.5 wt. % of the total weight of the composition. It is to be understood that these one or more essential oils providing an aromatherapy benefit may be used with our without the optional fragrancing constituent recited previously and may be used wholly or partially in place of said fragrancing constituent.

The largely aqueous topical antimicrobial cleaning compositions may include one or more antioxidant constituents; certain of these antioxidant constituents may additionally provide an anti-wrinkling benefit to the skin or other topical treatment benefit. Examples of antioxidants include but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, glutathione, and ascorbic acid and ascorbic acid derivatives (e.g., ascorbyl palmitate and ascorbyl polypeptide), as well as oil-soluble antioxidants such as butylated hydroxytoluene, retinoids, tocopherols e.g., tocopherol acetate, tocotrienols, and ubiquinone, natural extracts containing antioxidants such as extracts containing flavonoids and isoflavonoids and their derivatives, extracts containing resveratrol and the like, as well as certain natural extracts e.g., grape seed, green tea, pine bark, propolis, and the like. When present the total amount of such antioxidants are usually not in excess of 5%wt, preferably from 0.0001 - 4%wt. based on the total weight of the largely aqueous topical antimicrobial cleaning compositions of which it forms a part. In certain preferred embodiments one or more antioxidants constituents are necessarily present.

Optionally the largely aqueous topical antimicrobial cleaning compositions may include one or more vitamins. Examples of vitamins which can be added include vitamin A, such as vitamin A oil, retinol, retinyl acetate and retinyl palmitate; vitamin B, including vitamin B₂ such as riboflavin, riboflavin butyrate and flavin adenine nucleotide, vitamin B₆ such as pyridoxine hydrochloride, pyridoxine dioctanoate and pyridoxine tripalmitate, vitamin B₁₂ and its derivatives, and vitamin B₁₅ and its derivatives; vitamin C, such as L-ascorbic acid, L-ascorbic acid dipalmitic ester, sodium (L-ascorbic acid)-2-sulfate and dipotassium L-ascorbic acid diphosphate; vitamin D, such as ergocalciferol and cholecarciferol; vitamin E, such as alpha-tocopherol, beta-tocopherol, gamma-tocopherol, dl-alpha-tocopheryl acetate, dl-alpha-tocopheryl nicotinate and dl-alpha-tocopheryl succinate. When present, in accordance with certain of the preferred embodiments, one or more vitamins may be included in effective amounts, advantageously from 0.0001 - 1%wt., preferably from 0.001 - 0.75%wt. based on the total weight of the largely aqueous topical antimicrobial cleaning compositions of which it forms a part.

The inventive compositions may include one or more light stabilizers as well as UV absorbers or sunscreen constituents. Such materials are known to be useful in cosmetic or topical compositions and impart a degree of stability to the compositions which may comprise one or more components which may be deleteriously affected when exposed to certain sources of light, e.g., sunlight, fluorescent light sources. Other such materials are known to stabilize or improve the effect of colorants which may be present in the compositions. Any cosmetically acceptable material or compound which provides protection for one or more of the constituents in the inventive compositions from photolytic degradation or photo-oxidative degradation may be used. Examples include: triazines including s-triazine, triazine derivatives e.g. 2,4,6-trianilino-(p-carbo-2'-ethyl-1'-hexyloxy)-1,3,5-triazine, anisotriazine, ethylhexyltriazone, diethylhexylbutamidotriazone,; benzotriazoles and derivatives; esters of benzalmalonic acid; sulphonic acid derivatives of 3-benzylidencamphen; cinnamic acid and cinnamic acid amides, esters of cinnamonic acid; propane-1,3-diones; phenylbenzimidazoles and sulfonated benzimidazoles; salicylic acid derivatives including esters of salicylic acid, e.g., ethylhexyl salicylate, dipropylene glycol salicylate, TEA salicylate, salicylic acid 2-ethylhexylester, salicylic acid 4-isopropyl benzylester, salicylic acid homomenthylester; compounds or derivatives of compounds based on benzylidenecamphor, and the like. Any of the foregoing materials provided as acids may used in free acid form or as a salt thereof, e.g., an alkali, alkaline earth, ammonium, alkylammonium, alkanolammonium salt form thereof. When present, the one or more light stabilizers as well as UV absorbers may be included in any effective amount; advantageously such materials are present in amounts of from 0.0001 - 1%wt., preferably from 0.001 - 0.5%wt. based on the total weight of the composition of which it forms a part.

The inventive compositions may comprise an opacifier constituent. Such are materials which are typically emulsions, dispersions or suspensions of a water insoluble polymer or copolymer in a carrier. Nonlimiting examples of such opacifiers include commercially available products, such as latexes presently commercially available under the trademark ACUSOL (ex. Rohm & Haas Inc.). which are characterized by pH of about 2 to about 3, having approximately 40% solids in water, with particle size of about 0.1 to about 0.5 micron. Further preferred latexes useful in the present invention include those styrene/polyvinylpyrrolidone co-polymers and styrene/acrylic emulsions. Such include styrene/polyvinylpyrrolidone co-polymers which can be used include, for example, POLECTRON 430 (ex. ISP Technologies, Inc.), as well as styrene/acrylamide emulsion such as those marketed under the trade name OPULYN (ex. Rohm & Haas Inc.). When present, such opacifiers are advantageously included in generally minor amounts such as from 0.001 - 1 %wt. but desirably are present in amounts from 0.01 - 0.5%wt. Particularly preferred opacifiers are those which exclude anionic compounds or materials which may deleteriously interact with any of the cationic compounds present in the composition.

In order to adjust the pH of the inventive compositions, one or more pH adjusting agents as well as one or more pH buffers may optionally be included in the largely aqueous topical antimicrobial cleaning compositions in effective amounts. By way of non-limiting example pH adjusting agents include phosphorus containing compounds, monovalent and polyvalent salts such as of silicates, carbonates, and borates, certain acids and bases, tartrates and certain acetates. Further exemplary pH adjusting agents include mineral acids, basic compositions, and organic acids, which are typically required in only minor amounts. By way of further non-limiting example pH buffering compositions include the alkali metal phosphates, polyphosphates, pyrophosphates, triphosphates, tetraphosphates, silicates, metasilicates, polysilicates, carbonates, hydroxides, and mixtures of the same. Certain salts, such as the alkaline earth phosphates, carbonates, hydroxides, can also function as buffers. It may also be suitable to use as buffers such materials as aluminosilicates (zeolites), borates, aluminates and certain organic materials such as gluconates, succinates, maleates, and their alkali metal salts. When present, the pH adjusting agent, especially the pH buffers are present in an amount effective in order to maintain the pH of the inventive composition within a desired or a target pH range. Advantageously they may be included in generally minor amounts such as from 0.001 - 1.5 %wt. but desirably are present in amounts from 0.01 - 1%wt. Exemplary and preferred pH buffers and pH adjusting agents are described with reference to one or more of the following Examples.

The inventive largely aqueous topical antimicrobial cleaning compositions may include one or more chelating agents. Exemplary useful chelating agents include those known to the art, including by way of non-limiting example; aminopolycarboxylic acids and salts thereof wherein the amino nitrogen has attached thereto two or more substituent groups. Preferred chelating agents include acids and salts, especially the sodium and potassium salts of ethylenediaminetetraacetic acid, diethylenetriaminepentaacetic acid, N-hydroxyethylethylenediaminetriacetic acid, and of which the sodium salts of ethylenediaminetetraacetic acid may be particularly advantageously used. Such chelating agents may be omitted, or they may be included in generally minor amounts such as from 0.001 - 0.5 %wt. based on the weight of the chelating agents and/or salt forms thereof. Desirably, such chelating agents are included in the present inventive composition in amounts from 0.01 - 0.5%wt., but are most desirably present in reduced weight percentages from about 0.01 - 0.2%wt.

In a further aspect, the present invention also contemplates a method for providing a cleaning and/or providing an germicidal benefit to skin or other topical surface which method contemplates the topical application of the aqueous largely aqueous topical antimicrobial cleaning compositions as described herein in a cleaning and/or germicidally effective amount. Preferably according to the foregoing method, a germicidal benefit is provided to the skin or other topical surface to which the composition has been applied. Preferred embodiments of the largely aqueous topical antimicrobial cleaning compositions exhibit good germicidal efficacy of undesired microorganisms, e.g., *S.aureus, E.coli, P.aurugihosa,* as well as *E.hirae* on dermal (viz., skin. body) surfaces. Advantageously the largely aqueous topical antimicrobial cleaning compositions exhibit antimicrobial efficacy against one or more of certain gram positive pathogens, certain gram negative pathogens, certain viruses, certain fungi and/or certain mold.

While the largely aqueous topical antimicrobial cleaning compositions disclosed herein find a primary use in application to the skin to provide a cleaning and/or germicidal benefit thereto and is contemplated as being provided in a dispenser for use in such a treatment, it is to be understood that this is not to be understood as a limiting definition and that other forms and other uses of the present inventive composition, such as face lotion, milky lotion, massage materials, liquid toilet soap, as well as in hair care products such as shampoo, rinse or other hair or scalp treatment are expressly contemplated as being within the scope of the present invention. The largely aqueous topical antimicrobial cleaning compositions of the invention are beneficially formulated as a pourable lotion, a cosmetic milk, a liquid or a spray, but may also be formulated in transparent, translucent or opaque forms. In certain preferred embodiments the largely aqueous topical antimicrobial cleaning compositions is provided as a translucent or opaque composition.

The composition can be packaged in a suitable container to suit its viscosity and intended use by the consumer. For example, a lotion or cream can be packaged in a bottle, or can be packaged with a propellant in a propellant-driven aerosol device or alternately may be packaged in a container fitted with a manually operable pump. Advantageously the compositions which have low viscosities may be provided in bottles or flasks from which they be dispensed by pouring, or by pumping such as via a manually pumpable trigger pump or manually operable trigger spray pump. The inventive composition can be provided and stored in a non-deformable bottle but more preferably is provided in a squeezable container, such as a tube or deformable bottle which provides for easy dispensing of the composition by the consumer. Thus a further aspect of the invention provides a closed container containing the inventive composition as described herein.

The inventive compositions may also be dispensed from a motor driven pump or other dispensing device which may be adapted to dispense a dose or a continuous amount of the treatment composition from a container or refill or reservoir through the pump or dispensing device responsive to a suitable control signal or condition. Preferred, albeit non-limiting examples of such a dispensing device are described and illustrated in one or more of the following published documents: PCT/GB2009/002678; the contents of each of which are herein incorporated by reference.

It is to be further expressly understood that topical application of the largely aqueous topical antimicrobial cleaning compositions disclosed herein may be applied to the skin on any part of the body, including the skin on the face, neck, chest, back, arms, axilla, hands, legs, and scalp. The largely aqueous topical antimicrobial cleaning compositions disclosed herein may also be used on the hair. Preferably the largely aqueous topical antimicrobial cleaning compositions are not ingested or used on mucous tissues.

It is contemplated that in use, the consumer dispenses a quantity of the largely aqueous topical antimicrobial cleaning composition described herein and applied it to the skin or any other part of the body where they may be retained upon but are beneficially rubbed into the applied skin or other part of the body by the consumer to provide both a skin moisturization benefit concurrently with a germicidal benefit to the treated skin or other part of the body. Advantageously the thus applied largely aqueous topical antimicrobial cleaning composition is allowed to remain on the skin or other part of the body to which it has been applied, without any subsequent washing or rinsing. However, if desired by a consumer, the topical germicidal treatment compositions may be rinsed by the consumer under a stream of running water, e.g, in a shower or by immersion into water, e.g, a bath. Thus, a further aspect of the invention is directed to the use of the largely aqueous topical antimicrobial cleaning compositions as described herein.

In certain preferred embodiments the present inventive compositions comprise (consist of, or consist essentially of): highly aqueous, antimicrobially effective topical compositions which include:
at least one first cationic surfactant or salt thereof having antimicrobial or germicidal properties or salt form thereof;
at least one different, second quaternary ammonium cationic surfactant or salt form thereof according to the structure (I) 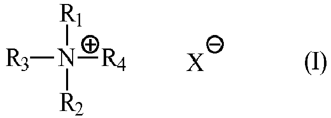 wherein:
R₁ is a C₂₀ - C₃₆, preferably unsubstituted alkyl moiety which may optionally be branched but is preferably unbranched,
R₂, R₃ and R₄ are independently C₁-C₃ alkyl moieties, and,
X is a salt forming counterion which renders the second quaternary surfactant water soluble or water dispersible, and,
   at least one amine oxide nonionic surfactant which is the predominant further surfactant in the composition with at least one nonionic surfactant based on an alkanolamide compound; which is preferably based on a mono- or di-alkanolamide especially preferably coconut monoethanolamide and/or lauramide diethanolamide;
   at least one polyethylene glycol acid ester and/or polypropylene glycol acid ester, preferably a polyethylene glycol distearate (150 EO),
   optionally but preferably a minor amount of at least one organic solvent, preferably a glycol ether;
   a pH adjusting agent and/or a pH buffer, and,
   water;
wherein the compositions are in the pH range of 4.5 - 5.5 and which compositions are preferably low viscosity, pourable unpressurized liquids which are used as cleansing compositions for topical surfaces.

The following examples below illustrate exemplary formulations as well as preferred embodiments of the invention. It is to be understood that these examples are provided by way of illustration only and that further useful formulations falling within the scope of the present invention and the claims may be readily produced by one skilled in the art without deviating from the scope and spirit of the invention.

### Examples

A number of largely aqueous topical antimicrobial cleaning compositions were produced according to the process described below, are described on Table 1 below. In the following compositions, the constituents were used "as supplied" from their respective suppliers and may constitute less than 100%wt. "actives", or may have been supplied as constituting 100%wt. "active" of the named compound, as indicated in the following Tables 1 and 2. The amounts indicated on Table 1 refer to %wt. of the "as supplied" named constituent used in a composition, as supplied by the corresponding constituent listed on Table 2. To each of the compositions was included deionized water in "quantum sufficient" (q.s.) in order to provide 100 parts by weight of the specific composition.

Compositions which are comparative examples are identified by digits preceded with the letter "C", while compositions according to the invention are identified by digits preceded with the letter "E".

| Table 1 | | |
|---|---|---|
| | 1755-192 | 1817-006 |
| | **C1** | **C2** |
| PEG-150 distearate (100%) | -- | -- |
| lauramide DEA (73%) | -- | -- |
| coconut monoethanolamide (100%) | -- | -- |
| cetrimonium chloride (30%) | 3.5 | -- |
| benzalkonium chloride (50%) | 0.26 | 0.26 |
| cocoamidopropyl hydroxysultaine | 7.0 | 7.0 |
| decyl glucoside | 2.0 | 2.0 |
| lauryl dimethyl amide oxide (30%) | -- | -- |
| glycerin (100%) | 2.0 | 2.0 |
| di-PPG-2 myreth-10 adipate | 0.1 | 0.1 |
| propylene glycol (100%) | 0.1 | 0.1 |
| preservative | 0.02 | 0.02 |
| tetrasodium EDTA (100%) | 0.2 | 0.2 |
| citric acid (50%) | q.s. | q.s. |
| fragrance | 0.22 | 0.22 |
| DI water (100%) | q.s. | q.s. |
| pH | 5.0 | 5.0 |
| viscosity (cPs, Brookfield Type LV3 viscometer, recommended spindle, at 12 rpm, sample at 20°-22°C) | n.t.* | n.t.* |
| %amine oxide/all surfactants (other than cationic surfactants), active weight basis | 0% | 0% |

| | | |
|---|---|---|
| n.t.* - while the indicated formulations were not tested, during the production of the samples the compositions were visually observed to be 'water thin', having a very thin viscosity approximate to that of deionized water. citric acid was added in a "quantum sufficient" in order to adjust the composition to the desired pH. | | |

| Table 1 | | | | | | | | | |
|---|---|---|---|---|---|---|---|---|---|
| | **E1** | **E2** | **E3** | **E4** | **E5** | **E6** | **E7** | **E8** | **E9** |
| PEG-150 distearate (100%) | 0.8 | 1.6 | 1.6 | 1.6 | 1.4 | 1.0 | 1.0 | 1.0 | 1.0 |
| lauramide diethanolamide (73%) | 0.8 | -- | 1.6 | 1.6 | -- | -- | -- | -- | -- |
| coconut monoethanolamide (100%) | -- | -- | -- | -- | 0.1 | 0.1 | 0.1 | 0.1 | 0.1 |
| cetrimonium chloride (30%) | 5.0 | 2.5 | 2.5 | 7.5 | 3.0 | 3.0 | 3.0 | 3.0 | 3.0 |
| benzalkonium chloride (50%) | 0.13 | 0.13 | 0.13 | 0.13 | 0.2 | 0.2 | 0.2 | 0.2 | 0.2 |
| lauryl dimethyl amide oxide (30%) | 5.0 | 10 | 10 | 10 | 5.0 | 5.0 | 5.0 | 5.0 | 5.0 |
| glycerin (100%) | -- | -- | -- | -- | 2.0 | 2.0 | 2.0 | 2.0 | 2.0 |
| propylene glycol (100%) | -- | -- | -- | -- | 0.5 | 0.5 | 0.5 | 0.5 | 0.5 |
| preservative | 0.02 | -- | -- | -- | 0.02 | 0.02 | 0.02 | 0.02 | 0.02 |
| tetrasodium EDTA (100%) | 0.15 | 0.3 | -- | 0.3 | 0.2 | 0.2 | 0.2 | 0.2 | 0.2 |
| citric acid (50%) | -- | -- | -- | -- | 0.45 | 0.23 | 0.23 | 0.23 | 0.23 |
| fragrance | -- | -- | -- | -- | 0.22 | 0.30 | 0.40 | 0.40 | 0.20 |
| colorant | -- | -- | -- | -- | -- | 0.0006 | 0.0005 | 0.0005 | 0.004 |
| di water (100%) | q.s. | q.s. | q.s. | q.s. | q.s. | q.s. | q.s. | q.s. | q.s. |
| pH | 4.79 | 4.82 | 4.82 | 4.81 | 5.36 | 4.8-5.5 | 4.8-5.5 | 4.8-5.5 | 4.8-5.5 |
| viscosity (cPs, Brookfield Type LV3 viscometer, recommended spindle (#1), at 60 rpm, sample at 20°-22°C) | n.t.* | n.t * | n.t.* | n.t.* | 5-50 | 5-50 | 5-50 | 5-50 | 5-50 |
| %amine oxide/all surfactants (other than cationic surfactants), active weight basis | 72.1% | 100% | 71.9% | 71.9% | 96.7% | 93.7% | 93.7% | 93.7% | 93.7% |

| | | | | | | | | | |
|---|---|---|---|---|---|---|---|---|---|
| n.t.* - while the indicated formulations were not quantitatively tested, during the production of the samples the compositions were visually observed to be 'water thin', having a very thin viscosity which was approximate to that of deionized water. | | | | | | | | | |

| Table 1 | | | | |
|---|---|---|---|---|
| | **E10** | **E11** | **E12** | **E13** |
| PEG-150 distearate (100%) | 1.0 | 1.0 | 1.0 | 1.0 |
| lauramide diethanolamide (73%) | - | - | - | - |
| coconut monoethanolamide (100%) | 0.1 | 0.1 | 0.1 | 0.1 |
| cetrimonium chloride (30%) | 3.0 | 3.0 | 3.0 | 3.0 |
| benzalkonium chloride (50%) | 0.2 | 0.2 | 0.2 | 0.2 |
| lauryl dimethyl amide oxide (30%) | 5.0 | 5.0 | 5.0 | 5.0 |
| glycerin (100%) | 2.0 | 2.0 | 2.0 | 2.0 |
| propylene glycol (100%) | 0.5 | 0.5 | 0.5 | 0.5 |
| preservative | 0.02 | 0.02 | 0.02 | 0.02 |
| tetrasodium EDTA (100%) | 0.2 | 0.2 | 0.2 | 0.2 |
| citric acid (50%) | 0.23 | 0.23 | 0.23 | 0.23 |
| fragrance** | 0.30 | 0.40 | 0.40 | 0.20 |
| colorant | 0.00062 | 0.00058 | 0.00057 | 0.00040 |
| di water (100%) | q.s. | q.s. | q.s. | q.s. |
| pH | 4.8-5.5 | 4.8-5.5 | 4.8-5.5 | 4.8-5.5 |
| viscosity (cPs, Brookfield Type LV3 viscometer, recommended spindle (#1), at 60 rpm, sample at 20°-22°C) | 5-50 | 5-50 | 5-50 | 5-50 |
| %amine oxide/all surfactants (other than cationic surfactants), active weight basis | 93.7% | 93.7% | 93.7% | 93.7% |

| | | | | |
|---|---|---|---|---|
| n.t.* - while the indicated formulations were not quantitatively tested, during the production of the samples the compositions were visually observed to be 'water thin', having a very thin viscosity which was approximate to that of deionized water. fragrance** - the identity of the fragrance constituent used in E10 - E13 was different for each of these example compositions. | | | | |

The constituents used to produce the compositions of Table 1 were as follows.

| Table 2 | |
|---|---|
| constituent | supplied by, (%wt. actives), source/supplier |
| PEG-150 distearate (100%) | Hallstar PEG 6000DS (100%wt. actives) ex. Hallstar |
| lauramide diethanolamide (73%) | Ninol 55-LL (73%wt. actives) ex. |
| coconut monoethanolamide (100%) | Mackamine CMA (100%wt. actives) ex. Rhodia |
| cetrimonium chloride (30%) | Ammonyx CETAC-30 (30%wt. actives) ex. Stepan co. |
| benzalkonium chloride (50%) | Barquat MB50 (50%wt. actives) ex. Lonza |
| lauryl dimethyl amine oxide (30%) | Ammonyx LO (30%wt. actives) ex. Stepan co. |
| glycerin (100%) | glycerin (100%wt. actives) USP grade |
| propylene glycol | propylene glycol (100%wt. actives), USP grade, ex. Dow |
| preservative | Kathon CG (used "as supplied") ex. Rohm & Haas |
| tetrasodium EDTA | Trilon B (100%wt. actives) ex. BASF |
| citric acid (50%) | aqueous dilution of technical grade citric acid (50%wt. actives) |
| fragrance | proprietary composition of its respective supplier |
| colorant | aqueous dispersion of one or more of: D&C Green #5, FD&C Red #33, FD&C Yellow #5, D&C Orange #4, FD&C Red #4 |
| di water | deionized water |

The compositions according to the invention may be produced by simple mixing of the constituents in order to produce a homogenous composition.

A preferred method of production includes the following steps:
a) Approximately 50% of the total amount of water is charged to a beaker (or other suitable vessel) into which is provided a motorized stirrer which is activated to ask take the contents of the beaker. The water is heated to 60-65°C after which is added (when present) the chelating agent, the PEG-150 distearate, and the alkanolamide nonionic surfactant (coconut monoethanolamide or lauramide diethanolamide) and stirring continues for approximately 10 - 30 minutes until the mixture within the beaker is fully heated, and is homogenous.
b) Thereafter, the heat source is removed or deactivated, and the remaining amount of water is added to the beaker on the stirring conditions in order to cool the contents of the beaker. Thereafter, are added under stirring conditions the second cationic compound (cetrimonium chloride), followed by the amine oxide surfactant, and followed by the glycerin (if present). Stirring continues until the contents of the beaker cool to approximately 30-35°C.
c) Subsequently, under stirring conditions are added the first cationic compound, any fragrance constituent (if present), followed by the remaining constituents which are added to the composition, e.g., preservatives, colorants and the like. Finally, sufficient amount of a pH adjusting agent, e.g., 50% aqueous solution of citric acid, is added in order to provide a desired product pH. Stirring continues until at least a homogenous mixture is formed, and advantageously an additional 10 - 30 of stirring is provided. Thereafter, the formed treatment composition can be removed from the beaker and may be used.

### Antimicrobial Efficacy (I) - EN1276 test:

The antimicrobial efficacy of certain compositions of Table 1 were evaluated in accordance with the EN1276 Standard Suspension Test for bactericidal activity. Samples of each test composition were placed in test tube with 3% BSA (dirty conditions) and challenge organism (to achieve 80% v/v) for a contact time of 1 minute, followed by neutralization, serial dilutions, plating and incubation. The results of the tested compositions are reported on the following Tables 3A-3E The acceptance criteria ("success criteria") for the purposes of the study was at least a 3 x log₁₀ reduction (99.9%) in 60 seconds of one or more of the challenge microorganisms.

| Table 3A - tested composition, E1 | | | | | | | | |
|---|---|---|---|---|---|---|---|---|
| Organism | S. *aureus* | | *E. coli* | | *P. aeruginosa* | | *E. hirae* | |
| Inoculum Level | 7.69 | | 7.66 | | 7.67 | | 7.68 | |
| Replicate | 1 | 2 | 1 | 2 | 1 | 2 | 1 | 2 |
| Recovery | < 2.18 | < 2.18 | < 2.18 | < 2.18 | < 2.18 | < 2.18 | < 2.18 | < 2.18 |
| Log₁₀ Reduction | > 5.51 | > 5.51 | > 5.48 | > 5.48 | > 5.49 | > 5.49 | > 5.50 | > 5.50 |
| Average Log₁₀ Reduction | > 5.51 | | > 5.48 | | > 5.49 | | > 5.50 | |
| % eradication | > 99.999 | | > 99.999 | | > 99.999 | | > 99.999 | |

| Table 3B - tested composition, E2 | | | | | | | | |
|---|---|---|---|---|---|---|---|---|
| Organism | *S*. *aureus* | | *E. coli* | | *P. aeruginosa* | | *E. hirae* | |
| Inoculum Level | 7.65 | | 7.69 | | 7.50 | | 7.68 | |
| Replicate | 1 | 2 | 1 | 2 | 1 | 2 | 1 | 2 |
| Recovery | 2.60 | 2.52 | < 2.18 | < 2.18 | < 2.18 | < 2.18 | < 2.18 | < 2.18 |
| Log₁₀ Reduction | 5.05 | 5.13 | > 5.51 | > 5.51 | > 5.32 | > 5.32 | > 5.50 | > 5.50 |
| Average Log₁₀ Reduction | 5.09 | | > 5.51 | | > 5.32 | | > 5.50 | |
| % eradication | 99.999 | | > 99.999 | | > 99.999 | | > 99.999 | |

| Table 3C - tested composition, E3 | | | | | | | | |
|---|---|---|---|---|---|---|---|---|
| Organism | S. *aureus* | | *E. coli* | | *P. aeruginosa* | | *E. hirae* | |
| Inoculum Level | 7.65 | | 7.69 | | 7.50 | | 7.68 | |
| Replicate | 1 | 2 | 1 | 2 | 1 | 2 | 1 | 2 |
| Recovery | 2.90 | 2.66 | < 2.18 | < 2.18 | < 2.18 | < 2.18 | < 2.18 | < 2.18 |
| Log₁₀ Reduction | 4.75 | 4.99 | > 5.51 | > 5.51 | > 5.32 | > 5.32 | > 5.50 | > 5.50 |
| Average Log₁₀ Reduction | 4.87 | | > 5.51 | | > 5.32 | | > 5.50 | |
| % eradication | 99.99 | | > 99.999 | | > 99.999 | | > 99.999 | |

| Table 3D - tested composition, E4 | | | | | | | | |
|---|---|---|---|---|---|---|---|---|
| Organism | *S. aureus* | | *E. coli* | | *P. aeruginosa* | | *E. hirae* | |
| Inoculum Level | 7.69 | | 7.66 | | 7.67 | | 7.68 | |
| Replicate | 1 | 2 | 1 | 2 | 1 | 2 | 1 | 2 |
| Recovery | < 2.18 | < 2.18 | < 2.18 | < 2.18 | < 2.18 | < 2.18 | < 2.18 | < 2.18 |
| Log₁₀ Reduction | > 5.51 | > 5.51 | > 5.48 | > 5.48 | > 5.49 | > 5.49 | > 5.50 | > 5.50 |
| Average Log₁₀ Reduction | > 5.51 | | > 5.48 | | > 5.49 | | > 5.50 | |
| % eradication | > 99.999 | | > 99.999 | | > 99.999 | | > 99.999 | |

| Table 3E - tested composition: E5 | | | | | | | | |
|---|---|---|---|---|---|---|---|---|
| Organism | *S. aureus* | | *E. coli* | | *P. aeruginosa* | | *E. hirae* | |
| Inoculum Level | 7.67 | | 7.67 | | 7.67 | | 7.66 | |
| Replicate | 1 | 2 | 1 | 2 | 1 | 2 | 1 | 2 |
| Recovery | < 2.18 | < 2.18 | < 2.18 | < 2.18 | < 2.18 | < 2.18 | < 2.18 | < 2.18 |
| Log₁₀ Reduction | > 5.49 | > 5.49 | > 5.49 | > 5.49 | > 5.49 | > 5.49 | > 5.48 | > 5.48 |
| Average Log₁₀ Reduction | > 5.49 | | > 5.49 | | > 5.49 | | > 5.48 | |
| % eradication | > 99.999 | | > 99.999 | | > 99.999 | | > 99.999 | |

As is seen from the foregoing the compositions according to the invention exhibited excellent antimicrobial efficacy even when the amounts of the cationic surfactant compounds are reduced as compared to the comparative example compositions.

The compositions of Table 1 all exhibited good foaming characteristics and good hand feel when used as a hand cleaning composition.

In contrast thereto, the compositions of C1 and C2 did not meet the foregoing acceptance criteria; the C1 and C2 compositions when tested with the foregoing protocol and exhibited an % eradication of one or more of: *E. coli, S. aureus, P. aerugihosa,* and *E. hirae* of < 90%., and thus did not meet the requirements of the success criteria.

### Antimicrobial Efficacy (II) - Minimum Inhibitory Concentration:

A Minimum Inhibitory Concentration evaluation of the example composition E11 was performed using a modification of the Macrodilution Broth Method outlined in CLSI Document M07-A8, Methods for Dilution Antimicrobial Susceptibility Tests for Bacteria that Grow Aerobically, Approved Standard, Eighth Edition. The tested composition was E11, which was challenged versus 27 different microorganism strains, 13 Gram-negative bacterial strains, 13 Gram-Positive bacterial strains, and one yeast species. Each challenge strain was exposed to each of 15 doubling dilutions of the E11 composition which was prepared in sterile nutrient broth. Following incubation, the Minimum Inhibitory Concentration of the test product was determined visually and documented.

The challenged microorganism strains were as follows:
*Acinetobacter baumannii* (ATCC #19606)
*Acinetobacter lwoffii* (ATCC #15309)
*Burkholderia cepacia* (ATCC#25416)
*Candida albicans* (ATCC #10231)
*Corynebacterium jeikeium* (ATCC # 43734)
*Enterobacter aerogenes* (ATCC #13048),
*Enterobacter cloacae cloacae* (ATCC #13047),
*Enterococcus faecalis* (ATCC #19433)
*Enterococcus faecalis* VRE (ATCC #51299)
*Enterococcus faecium* (ATCC # 19434)
*Escherichia coli* (ATCC #11229)
*Escherichia coli* (ATCC #25922)
*Haemophilus influenzae* (ATCC #19418)
*Klebsiella oxytoca* (ATCC #43165)
*Klebsiella pneumoniae ozaenae* (ATCC #11296)
*Micrococcus luteus* (ATCC #4698)
*Proteus mirabilis* (ATCC #7002)
*Pseudomonas aerugihosa* (ATCC #9027)
*Serratia marcescens* (ATCC #14756)
*Staphylococcus aureus aureus* (ATCC #6538)
*Staphylococcus aureus aureus* MRSA (ATCC #33592)
*Staphylococcus epidermidis* (ATCC #12228)
*Staphylococcus haemolyticus* (ATCC #29970)
*Staphylococcus hominis hominis* (ATCC # 27844)
*Staphylococcus saprophyticus* (ATCC #15305)
*Streptococcus pneumoniae* (ATCC #6303)
*Streptococcus pyogenes* (ATCC #19615)
MRSA= Methicillin-Resistant *Staphylococcus aureus*
VRE= Vancomycin-Resistant *Enterococcus*

The results of the testing are reported on the following Table 4:

| Table 4 | | | |
|---|---|---|---|
| Challenge Microorganism Species | ATCC # | Incoculum Population (per tube; CFU/mL) | Minimum Inhibitory Concentration (MIC) (Expressed As Product Dilution) |
| *Acinetobacter baumannii* | 19606 | 6.0250 x 10⁵ | 1:1,024 |
| *Acinetobacter Iwoffii* | 15309 | 9.3250 x 10⁵ | 1:8,192 |
| *Burkholderia cepacia* | 25416 | 9.550 x 10⁵ | 1:8 |
| *Candida albicans* | 10231 | 7.5750 x 10⁵ | 1:2,048 |
| *Corynebacterium jeikeium* | 43734 | 2.0250 x 10⁶ | 1:8,192 |
| *Enterobacter aerogenes* | 13048 | 8.350 x 10⁵ | 1:256 |
| *Enterobacter cloacae cloacae* | 13047 | 6.0750 x 10⁵ | 1:512 |
| *Enterococcus faecalis* | 19433 | 9.550 x 10⁵ | 1:8,192 |
| *Enterococcus faecalis* VRE | 51299 | 1.450 x 10⁶ | 1:2,048 |
| *Enterococcus faecium* | 19434 | 8.850 x 10⁵ | 1:4,096 |
| *Escherichia coli* | 11229 | 1.0825 x 10⁶ | 1:256 |
| *Escherichia coli* | 25922 | 5.850 x 10⁵ | 1:512 |
| *Haemophilus influenzae* | 19418 | 1.2925 x 10⁷ | 1:2,048 |
| *Klebsiella oxytoca* | 43165 | 7.20 x 10⁵ | 1:128 |
| *Klebsiella pneumoniae ozaenae* | 11296 | 8.0750 x 10⁵ | 1:2,048 |
| *Micrococcus luteus* | 4698 | 1.330 x 10⁶ | > 1:32, 768 |
| *Proteus mirabilis* | 7002 | 1.1125 x 10⁶ | 1:16 |
| *Pseudomonas aeruginosa* | 9027 | 7.3750 x 10⁵ | 1:8 |
| *Serratia marcescens* | 14756 | 6.20 x 10⁵ | 1:256 |
| *Staphylococcus aureus aureus* | 6538 | 1.3950 x 10⁶ | 1:8,192 |
| *Staphylococcus aureus aureus* MRSA | 33592 | 1.950 x 10⁶ | 1:2,048 |
| *Staphylococcus epidermidis* | 12228 | 8.950 x 10⁵ | 1:16,384 |
| *Staphylococcus haemolyticus* | 29970 | 4.2750 x 10⁵ | 1:16,384 |
| *Staphylococcus hominis hominis* | 27844 | 3.250 x 10⁵ | 1:16,384 |
| *Staphylococcus saprophyticus* | 15305 | 6.6750 x 10⁵ | 1:16,384 |
| *Streptococcus pneumoniae* | 6303 | 5.00 x 10⁶ | 1:2,048 |
| *Streptococcus pyogenes* | 19615 | 1.2925 x 10⁶ | 1:4,096 |

| | | | |
|---|---|---|---|
| MRSA= Methicillin-Resistant *Staphylococcus aureus* VRE= Vancomycin-Resistant *Enterococcus* | | | |

As can be seen from the reported test results of the foregoing table, the composition according to E11 exhibited excellent inhibitory activity, depending upon the microorganism strain evaluated.

### Antimicrobial Efficacy (III) - In-Vitro Time-Kill:

The antimicrobial efficacy of example composition E11 of Table 1 (diluted to 90%v/v in distilled water) was evaluated. Neutralization studies of the tested product was performed versus *Escherichia coli* (ATCC #11229), *Staphylococcus aureus* (ATCC #6538) and *Steptococcus pneumoniae* (ATCC #6303) to ensure that the neutralizing solution employed (Butterfield's Phosphate Buffer solution with production neutralizer [BBP ++ RB]) was effective in reducing the antimicrobial properties of the tested example composition and was non-toxic to each of the challenge microorganism strains used in the test. This neutralization procedure was based on ASTM E-1054-08, *Standard Test Methods for Evaluation of Inactivators of Antimicrobial Agents.* Under the conditions of this evaluation, the neutralizing solution used (BBP ++ RB) was demonstrated to be effective in neutralizing the antimicrobial properties of the tested E11 dilution, and was also shown to be non-toxic to all of the challenge microorganism strains used in the test.

The challenge microorganism strains used in the test were as follows:
*Burkholderia cepacia* (ATCC #25416)
*Enterococcus faecalis* (ATCC #51299)
*Esherichia coli* (ATCC #11229)
*Klebsiella pneumoniae pneumoniae* (ATCC #10031)
*Psuedomonas aeruginosa* (ATCC #9027)
*Serratia marcescens* (ATCC #14756)
*Staphylococcus aureus aureus* (ATCC #6538)
*Staphylococcus aureus aureus* MRSA(ATCC #33592)
*Staphylococcus epidermidis* (ATCC #12228)
*Streptococcus pneumoniae* (ATCC #6303)
*Streptococcus pyogenes* (ATCC #19615)
MRSA = Methicillin-Resistant *Staphylococcus aureus*

The following Table 5 reports the results of the test, and presents the Initial Population (colony forming units/milliliter = CFU/mL) of the specific challenge microorganism strain tested, the post-exposure population (CFU/mL), and the Log₁₀ as well as the percent reduction produced by the dilution of the E11 composition 90% v/v) at the times indicated.

| Table 5 | | | | | |
|---|---|---|---|---|---|
| Challenge Microorganism | Inoculum Level (CFU/mL | Exposure Time | Post-Exposure Population (CFU/mL) | Log₁₀ Reduction | Percent Reduction |
| *Burkholderia cepacia* (ATCC #25416) | 2.1550 x 10⁹ | 30 seconds | 4.850 x 10⁸ | 0.6477 | 77.4942% |
| | | 1 minute | 2.460 x 10⁸ | 0.9425 | 88.5847% |
| *Enterococcus faecalis* (ATCC #51299) | 4.550 x 10⁹ | 30 seconds | <1.00 x 10³ | 6.6580 | 99.9999% |
| | | 1 minute | <1.00 x 10³ | 6.6580 | 99.9999% |
| *Esherichia coli* (ATCC #11229) | 1.4650 x 10⁹ | 30 seconds | <1.00 x 10³ | 6.1658 | 99.9999% |
| | | 1 minute | <1.00 x 10³ | 6.1658 | 99.9999% |
| *Klebsiella pneumoniae pneumoniae* (ATCC #10031) | 8.750 x 10⁸ | 30 seconds | <1.00 x 10³ | 5.9420 | 99.9999% |
| | | 1 minute | <1.00 x 10³ | 5.9420 | 99.9999% |
| *Psuedomonas aeruginosa* (ATCC #9027) | 2.010 x 10⁹ | 30 seconds | <1.00 x 10³ | 6.3032 | 99.9999% |
| | | 1 minute | <1.00 x 10³ | 6.3032 | 99.9999% |
| *Serratia marcescens* (ATCC #14756) | 8.10 x 10⁸ | 30 seconds | 2.130 x 10⁷ | 1.5801 | 97.3704% |
| | | 1 minute | 1.680 x 10⁶ | 2.6832 | 99.7926% |
| *Staphylococcus aureus aureus* (ATCC #6538) | 1.30 x 10⁹ | 30 seconds | 9.00 x 10³ | 5.1597 | 99.9993% |
| | | 1 minute | <1.00 x 10³ | 6.1139 | 99.9999% |
| *Staphylococcus aureus aureus* MRSA (ATCC #33592) | 1.750 x 10⁹ | 30 seconds | 1.1350 x 10⁵ | 4.1880 | 99.9935% |
| | | 1 minute | 5.50 x 10³ | 5.5026 | 99.9997% |
| *Staphylococcus epidermidis* (ATCC #12228) | 7.650 x 10⁸ | 30 seconds | <1.00 x 10³ | 5.8837 | 99.9999% |
| | | 1 minute | <1.00 x 10³ | 5.8837 | 99.9999% |
| *Streptococcus pneumoniae* (ATCC #6303) | 5.350 x 10⁹ | 30 seconds | <1.00 x 10⁴ | 5.7284 | 99.9998% |
| | | 1 minute | <1.00 x 10⁴ | 5.7284 | 99.9998% |
| *Streptococcus pyogenes* (ATCC #19615) | 1.0750 x 10⁹ | 30 seconds | <1.00 x 10³ | 6.0314 | 99.9999% |
| | | 1 minute | <1.00 x 10³ | 6.0314 | 99.9999% |

As can be seen from the reported test results of the foregoing table, the diluted composition according to E11 exhibited excellent activity, which varied only slightly depending upon the microorganism strain evaluated.

### Antimicrobial Efficacy (IV) - Handwash Test:

The antimicrobial efficacy of example composition E11 of Table 1 was evaluated as to its efficacy in the reduction of a challenge microorganism which was evaluated generally in accordance with a test protocol based on 1994 FDA TFM, Health-Care Antiseptic Drug Products; Proposed Rule.

According to the test protocol, as test subjects were selected fifteen overtly healthy persons at least 18 years of age. All subjects' hands were free from clinically evident dermatoses, injuries to the hands or forearms, hangnails, and/or any other disorders that may have compromised the subject or the study. The test subjects first were subjected to a 7-day pre-test conditioning period followed by a single test day. On the test day, ten product applications of an aliquot of the E11 composition were applied. The test subjects used the E11 composition. over the course of 11 consecutive hand contaminations with an indicator microorganism, *Serratia marcescens* (ATCC #14756), the first followed by a sample for baseline population, and an additional 10 by product applications 1, 3, 7, and 10. Hand-sampling was performed using the Glove Juice Sampling Procedure.

The 7 days prior to the test portion of the study constituted the pre-test period. During this time, the test subjects avoided the use of the medicated soaps, lotions, deoterants and shampoos, as well as skin contact with solvents, detergents, acids and bases, or any other skin products known to affect the normal microbial populations of the skin. Subjects were supplied a personal hygiene kit containing nonmedicated soap, shampoo, lotion, and rubber gloves to be worn when contact with antimicrobials, solvents, detergents, acids, or bases could not be avoided. Subjects were instructed to use the contents of this kit exclusively during their participation in the study. Subjects also avoided using UV tanning beds or sunbathing, and swimming or bathing in biocide-treated pools or hot tubs.

In preparation for the tests performed on the test day, an inoculum of the indicator microorganism, *Serratia marcescens* (ATCC #14756) was prepared for use to challenge the efficacy of the E11 composition.

A stock culture of *Serratia marcescens* (ATCC #14756) was prepared by aseptically transferring contents of a lyophilized vial to approximately 5.0 mL of sterile Tryptic Soy Broth (TSB), which was then incubated at 25 °C ± 2 °C for 20 hours. 2-L flasks containing approximately 1,000 mL TSB were inoculated with 1.0 mL of the 20-hour broth cultures, and incubated fro 21 - 26 hours at 25 °C ± 2 °C. Prior to any withdrawal of culture, whether for hand contamination or for numbers assay, the suspensions were stirred or swirled. The suspensions were assayed for number of organisms at the beginning and end of the use periods. Suspensions were not used for more than 8 hours.

On the test day, prior to being admitted into testing, subjects were questioned regarding their adherence to the Protocol requirements. Subjects clipped their fingernails to a free edge of ≤ 1 mm, if they had not already done so. All jewelry was removed from the hands and arms prior to washing. Using tap water, subjects performed a practice hand-inoculation procedure. A 5.0-mL aliquot of tap water was transferred into each subject's cupped hands in three volumes of approximately 1.5 mL, 1.5 mL, and 2 mL. A 30-second handwash using nonmedicated soap and a 30-second rinse were performed to remove dirt and oil from hands. The temperature of the water used for this and subsequent wash procedures was controlled at 40 °C ± 2 °C.

Thereafter, a 5.0-mL aliquot of a suspension containing approximately 1.0 x 10⁹ CFU/mL of *Serratia marcescens* (ATCC #14756) was transferred into each subjects cupped hands in three volumes of approximately 1.5 mL, 1.5 mL, and 2 mL. The inoculum distributed evenly over both hands, not reaching above the wrists, via gentle continuous massage for 45 seconds. After a timed 2-minute air-dry, and within 5 minutes after contamination for baseline, and/or after product applications 1, 3, 7, and 10, powder-free sterile latex gloves were placed on subjects' hands, and 75 mL of Stripping Suspending Fluid (SSF) were instilled into each of the gloves. The wrists were secured, and technicians massaged the hands through the gloves in a standardized manner for 60 seconds. Within 1 minute of completing the post-application massages, a 5-mL aliquot of the glove juice was removed from each of the gloves and diluted in 5.0 mL Butterfield's Phosphate Buffer Solution with product neutralizers (BBP++) (dilution 10°). The 10° dilutions were then serially diluted in BBP++. Duplicate spiral plates were prepared from specified dilutions on Tryptic Soy Agar with product neutralizers (TSA+) and incubated at 25 °C ± 2 °C for approximately 40 hours, when sufficient growth was observed. Colonies were counted and data recorded using the computerized QCOUNT™ plate-counting system.

The first contamination cycle provide the baseline recovery populations. It was followed with a 30-second handwash using nonmedicated soap and a 30-second rinse.

A 5.0-mL aliquot of the microbial inoculum was again evenly distributed over both hands, not reaching above the wrists, via gentle continuous massage for 45 seconds. After a timed 2-minute air-dry, the subjects applied 5 mL of the E11 composition which was e dispensed into the test subjects' cupped hands. Thereafter the test subjects rubbed hands together, lathering hands and forearms to just above the wrist for 30 seconds. Subjects rinsed for 30 seconds. The hands were gloved wet for sampling. If product application was not followed by a sample, the test subjects' dried their hands with a paper towel.

Each test subject completed this contamination/product application a total of 10 consecutive times, with a minimum of 5 minutes between contamination/product applications. The test subjects' hands were sampled for residual *Serratia marcescens* (ATCC #14756) after contamination/product application cycles 1, 3, 7, and 10. Sampling was performed after Applications 1, 3, 7, and 10.
All sampling was performed using the aforementioned protocol wherein powder-free sterile latex gloves were placed on subjects' hands, SSF was instilled, the wrists secured, the hands massaged, and within 1 minute thereafter a 5-mL aliquot removed, neutralized and serially diluted.

The performance of the E11 composition was evaluated post-application based upon mean log₁₀ reductions from baseline. Log₁₀ reductions were calculated by subtracting the log₁₀ number of bacteria recovered at the specified sampling times following product application (applications 1, 3, 7, and 10) from the log₁₀ number of bacteria recovered from the baseline-sampling. The critical index was a mean log₁₀ reduction in bacteria of ≥ 2 log₁₀ after the first application of the E11 composition. The Minitab^{®} computer package was used for all statistical calculations. Statistical calculations of mean, standard deviation, and 95% confidence intervals were performed on the log₁₀ microbial recoveries in baseline and post-product application samples, and the reductions from the baseline. The Log₁₀ microbial recoveries from each subject's hands and reductions from baseline populations are presented in the following Table 6 which provides the mean Log₁₀ microbial recoveries of *Serratia marcescens (ATCC #14756)* and the mean Log₁₀ reductions from the baseline following the use of the E11 composition, reported for each test subject, and each hand.

| Table 6 | | | | | | | | | | |
|---|---|---|---|---|---|---|---|---|---|---|
| Subject | Hand | Baseline Log₁₀ Microbial Recovery | Post-Wash 1 Log₁₀ Microbial Recovery | Post-Wash 3 Log₁₀ Microbial Recovery | Post-Wash 7 Log₁₀ Microbial Recovery | Post-Wash 10 Log₁₀ Microbial Recovery | Post-Wash 1 Log₁₀ Reduction from Baseline | Post-Wash 3 Log₁₀ Reduction from Baseline | Post-Wash 7 Log₁₀ Reduction from Baseline | Post-Wash 10 Log₁₀ Reduction from Baseline |
| 2 | Left | 9.00 | 6.74 | 6.28 | 5.81 | 5.74 | 2.26 | 2.72 | 3.19 | 3.26 |
| | Right | 8.89 | 6.50 | 5.97 | 5.45 | 5.92 | 2.39 | 2.91 | 3.44 | 2.97 |
| 4 | Left | 9.15 | 6.33 | 6.07 | 5.44 | 5.33 | 2.82 | 3.08 | 3.72 | 3.82 |
| | Right | 9.28 | 5.98 | 5.80 | 5.36 | 5.54 | 3.30 | 3.48 | 3.92 | 3.74 |
| 5 | Left | 9.27 | 6.86 | 6.37 | 5.94 | 5.79 | 2.41 | 2.90 | 3.33 | 3.48 |
| | Right | 9.31 | 6.45 | 6.21 | 5.80 | 5.51 | 2.86 | 3.10 | 3.51 | 3.81 |
| 6 | Left | 8.96 | 6.62 | 6.55 | 5.75 | 5.84 | 2.34 | 2.41 | 3.21 | 3.12 |
| | Right | 8.90 | 6.49 | 6.30 | 6.00 | 6.09 | 2.42 | 4.60 | 2.90 | 2.82 |
| 10 | Left | 9.34 | 6.05 | 5.62 | 5.10 | 5.18 | 3.30 | 3.72 | 4.24 | 4.16 |
| | Right | 9.24 | 6.38 | 5.41 | 4.91 | 4.82 | 2.85 | 3.83 | 4.33 | 4.42 |
| 11 | Left | 8.83 | 6.18 | 6.02 | 5.80 | 5.28 | 2.64 | 2.81 | 3.03 | 3.54 |
| | Right | 8.81 | 6.55 | 6.35 | 5.63 | 5.46 | 2.26 | 2.46 | 3.18 | 3.36 |
| 13 | Left | 9.36 | 6.80 | 6.48 | 6.16 | 6.05 | 2.56 | 2.88 | 3.20 | 3.31 |
| | Right | 9.38 | 6.42 | 6.33 | 6.10 | 5.87 | 2.96 | 3.05 | 3.29 | 3.51 |
| 17 | Left | 9.23 | 6.66 | 6.60 | 6.13 | 6.17 | 2.58 | 2.64 | 3.10 | 3.06 |
| | Right | 9.24 | 6.21 | 6.43 | 5.93 | 5.87 | 3.03 | 2.81 | 3.31 | 3.37 |
| 20 | Left | 9.24 | 6.66 | 6.25 | 6.01 | 5.99 | 2.58 | 2.99 | 3.22 | 3.25 |
| | Right | 9.24 | 6.33 | 6.02 | 5.65 | 5.62 | 2.91 | 3.22 | 3.59 | 3.62 |
| 21 | Left | 9.17 | 6.11 | * | 5.84 | 5.72 | 3.06 | * | 3.33 | 3.45 |
| | Right | 9.04 | 6.13 | * | 5.68 | 5.65 | 2.91 | * | 3.36 | 3.39 |
| 23 | Left | 8.98 | 6.12 | 6.06 | 5.64 | 5.57 | 2.86 | 2.91 | 3.34 | 3.40 |
| | Right | 9.01 | 6.35 | 5.92 | 5.46 | 5.34 | 2.66 | 3.09 | 3.55 | 3.67 |
| 26 | Left | 9.32 | 6.53 | 6.03 | ** | 5.46 | 2.79 | 3.29 | ** | 3.86 |
| | Right | 9.20 | 6.77 | 6.36 | 6.10 | 5.59 | 2.43 | 2.84 | 3.10 | 3.61 |
| 27 | Left | 9.33 | 6.37 | 5.66 | 5.53 | 5.47 | 2.97 | 3.67 | 3.80 | 3.87 |
| | Right | 9.34 | 6.17 | 5.81 | 5.39 | 5.41 | 3.17 | 3.53 | 3.95 | 3.93 |
| 28 | Left | 8.99 | 6.18 | 5.85 | 5.53 | 5.33 | 2.80 | 3.13 | 3.46 | 3.66 |
| | Right | 9.02 | 6.00 | 5.60 | 5.31 | 4.90 | 3.02 | 3.42 | 3.72 | 4.12 |
| 29 | Left | 9.06 | 5.94 | 6.01 | 5.18 | 5.78 | 3.12 | 3.05 | 3.88 | 3.29 |
| | Right | 9.07 | 6.10 | 5.56 | 5.31 | 5.40 | 2.97 | 3.52 | 3.76 | 3.68 |

| | | | | | | | | | | |
|---|---|---|---|---|---|---|---|---|---|---|
| * Data not used in analysis due to deviation from protocol ** Not used in analysis due to apparent error | | | | | | | | | | |

As can be understood with reference to the results reported on Table 6, the E11 composition produced a mean log₁₀ reduction of *Serratia marcescens* (ATCC#14756) of 2.77 after Application 1, indicating good efficacy against this indicator microorganism on hands of human test subjects.

### Antimicrobial Efficacy (V) - EN1276 test:

The antimicrobial efficacy of example composition E9 at three different dilutions (10% v/v, 50% v/v and 80% v/v, in distilled water) was evaluated in accordance with the EN1276 (:2012) Standard Suspension Test for bactericidal activity against *Staphylococcus aureus* (ATCC #6538), *Pseudomonas aeruginosa* (ATCC# 15442), *Escherichia coli* (ATCC #10536) and *Enterococcus hirae* (ATCC #10541). Samples of each test composition were placed in test tube with 3% BSA (dirty conditions) and challenge organism to achieve the indicated dilutions for a contact time of 1 minute, followed by neutralization, serial dilutions, plating and incubation. The test was performed at room temperature (approx. 20°C +/- 0.6 °C).The results of the tested compositions are reported on the following Table 7.

**Table 7**

| test strain | validation test | | | | microbial suspension in the test | | test procedure at product concentration V/V | | |
|---|---|---|---|---|---|---|---|---|---|
| | bacterial suspension | Test conditions | neutralizer control | dilution-neutralization control | | | | | |
| | | | | | | | 10% | 50 % | 80 % |
| | Vc: 115;139 | Vc: 119;126 | Vc:108;120 | Vc: 85;103 | 10⁻⁶: >330;>330 | Vc | >330;>330 | 0;0 | 0;0 |
| Pseudomonas aeruginosa | Nv₀: 1,3•10² | A: 1,2•10² | B: 1,1•10² | C: 9,4•10¹ | 10⁻⁷: 42;55 | Na | > 3300 | < 140 | < 140 |
| PCM 2563 = | | | | | N: 4,8•10⁸ | lg Na | > 3,52 | < 2,15 | < 2,15 |
| ATCC 15442 | | | | | N₀ 4,8•10⁷ | R | < 4,16 | > 5,53 | > 5,53 |
| | | | | | lgN₀=7,68 | | | | |
| | Vc: [03;110 | Vc: 127;144 | Vc: 98;114 | Vc: 89;96 | 10⁻⁶:>330;>330 | Vc | 8;13 | 5;6 | 15;21 |
| Staphylococcus aureus | Nv₀: 1,1•10² | A: 1,4•10² | B: 1,1•10² | C: 9,2•10¹ | 10⁻⁷: 39;46 | Na | < 140 | <140 | 180 |
| ATCC 6538 | | | | | N: 4,2•10³ | Ig Na | < 2,15 | < 2,15 | 2,26 |
| | | | | | N₀ 4,2•10⁷ | R | > 5,47 | > 5,47 | 5,36 |
| | | | | | IgN₀=7,62 | | | | |
| | Vc: 65;78 | Vc: 68;73 | Vc: 57;70 | Vc: 47;66 | 10⁻⁶: 254;271 | Vc | 0;0 | 0;0 | 0;0 |
| Escherichia coli | Nv₀: 7,2•10¹ | A: 7,0•10¹ | B: 6,4•10¹ | C: 5,6•10¹ | 10⁻⁷: 30,36 | Na | < 140 | <140 | < 140 |
| PCM 1144 = | | | | | N: 2,7•10⁸ | Ig Na | < 2,15 | <2,15 | <2,15 |
| ATCC 10536 | | | | | N₀ 2,7•10⁷ | R | > 5,28 | > 5,28 | > 5,28 |
| | | | | | lgN₀=7,43 | | | | |
| | Vc: 42;56 | Vc: 38;46 | Vc: 48;53 | Vc: 62;68 | 10⁻⁶: 174;198 | Vc | 0;0 | 0;0 | 0;0 |
| Enterococcus hirae | Nv₀: 4,9•10¹ | A: 4,2•10¹ | B: 5,0•10¹ | C: 6,5•10¹ | 10⁻⁷: 12;19 | Na | <140 | <140 | <140 |
| PCM 2559 = | | | | | N: 1,9•10⁸ | Ig Na | < 2,15 | <2,15 | <2,15 |
| ATCC 10541 | | | | | N₀ 1,9•10⁷ | R | > 5,13 | > 5,13 | > 5,13 |
| | | | | | lgN₀=7,28 | | | | |

| | | | | | | | | | |
|---|---|---|---|---|---|---|---|---|---|
| Vc - viable count Na - cfu/ml in the test mixture N - cfu/ml in the bacterial test suspension, N₀= N/10 A - cfu/ml in the experimental conditions control test Nv - cfu/ml in the bacterial suspension in validation procedure, Nv₀= Nv/10 B - cfu/ml in neutralizer control test R - reduction factor of viable counts C - cfu/ml in the dilution - neutralization, method control test Validation criteria verification: 1,5·10⁸<N < 5,0 ·10⁸ - met A,B,C > 0,5 Nv₀ - met 3,0·10²<Nᵥ<1,6·10³ - met | | | | | | | | | |

### Antimicrobial Efficacy (VI) - EN1276 test:

The antimicrobial efficacy of example composition E10 at three different dilutions (10% v/v, 50% v/v and 80% v/v, in distilled water) was evaluated in accordance with the EN1276 Standard Suspension Test for bactericidal activity against *Salmonella enterica* (ATCC #13311), *Staphylococcus aureus* (ATCC #33592), *Staphylococcus aureus* (ATCC #6538), *Klebsiella pneumoniae* (ATCC BAA 2146), *Pseudomonas aeruginosa* (ATCC# 15442), *Escherichia coli* (ATCC #10536) and *Enterococcus hirae* (ATCC #10541). Samples of each test composition were placed in test tube with 3% BSA (dirty conditions) and challenge organism to achieve the indicated dilutions for a contact time of 1 minute, followed by neutralization, serial dilutions, plating and incubation. The test was performed at room temperature (approx. 20°C +/- 0.6 °C).The results of the tested compositions are reported on the following Tables 8 and 9.

**Table 8**

| test strain | validation test | | | | microbial suspension in the test | | test procedure at product concentration V/V | | |
|---|---|---|---|---|---|---|---|---|---|
| | bacterial suspension | Test conditions | neutralizer control | dilution-neutralization control | | | | | |
| | | | | | | | 10 % | 50 % | 80 % |
| | Vc: 115;139 | Vc: 119;126 | Vc: 108;120 | Vc: 124;142 | 10⁻⁶: >330,>330 | Vc | >330;>330 | 0;0 | 0;0 |
| Pseudomonas aeruginosa | Nv₀: 1,3•10² | A: 1,2•10² | B; 1,1·10² | C: 1,3•10² | 10⁻⁷: 42;55 | Na | > 3300 | < 140 | < 140 |
| PCM 2563 = | | | | | N: 4,8•10⁸ | lg Na | > 3,52 | < 2,15 | < 2,15 |
| ATCC 15442 | | | | | No 4,8•10⁷ | R | < 4,16 | > 5,53 | > 5,53 |
| | | | | | Ig N₀=7.68 | | | | |
| | Vc: 103;110 | Vc: 127;144 | Vc:98;114 | Vc: 86;103 | 10⁻⁶: >330;>330 | Vc | 7;11 | 17;24 | 14;20 |
| Staphylococcus aureus | Nv₀: 1,1•10² | A: 1,4•10² | B: 1,1•10² | C: 9,4•10¹ | 10⁻⁷: 39;46 | Na | < 140 | 205 | 170 |
| ATCC 6538 | | | | | N: 4,2•10⁸ | lg Na | < 2,15 | 2,31 | 2,23 |
| | | | | | N₀ 4,2•10⁷ | R | > 5,47 | 5,31 | 5,39 |
| | | | | | lgN₀=7,62 | | | | |
| | Vc: 65;78 | Vc: 68;73 | Vc: 57;70 | Vc: 81;86 | 10⁻⁶; 254;271 | Vc | 0;0 | 0;0 | 0;0 |
| Escherichia coli | Nv₀: 7,2•10¹ | A: 7,0•10¹ | B: 6,4•10¹ | C: 8,4•10¹ | 10⁻⁷: 30;36 | Na | < 140 | < 140 | < 140 |
| PCM 1144 = | | | | | N: 2,7•10⁸ | lg Na | < 2,15 | < 2,15 | < 2,15 |
| ATCC 10536 | | | | | N₀ 2,7•10⁷ | R | > 5,28 | > 5,28 | > 5,28 |
| | | | | | lgN₀=7,43 | | | | |
| | Vc: 42;56 | Vc: 38;46 | Vc: 48;53 | Vc: 31;44 | 10⁻⁶: 174;198 | Vc | 5;12 | 0;0 | 1;5 |
| Enterococcus hirae | Nv₀: 4,9•10¹ | A: 4,2•10¹ | B: 5,0•10¹ | C: 3,8•10¹ | 10⁻⁷: 12;19 | Na | < 140 | < 140 | < 140 |
| PCM 2559 = | | | | | N: 1,9•10⁸ | lg Na | < 2,15 | < 2,15 | < 2,15 |
| ATCC 10541 | | | | | N₀ 1,9•10⁷ | R | > 5,13 | > 5,13 | > 5,13 |
| | | | | | lgN₀=7,28 | | | | |

| | | | | | | | | | |
|---|---|---|---|---|---|---|---|---|---|
| Vc - viable count Na - cfu/ml in the test mixture N - cfu/ml in the bacterial test suspension, N₀= N/10 A - cfu/ml in the experimental conditions control test Nv - cfu/ml in the bacterial suspension in validation procedure, Nv₀= Nv/10 B - cfu/ml in neutralizer control test R - reduction factor of viable counts C - cfu/ml in the dilution - neutralization method control test Validation criteria verification: 1,5·10⁶ < N < 5,0·10⁸ - met A,B,C > 0,5 Nv₀ - met 3,0·10² < Nᵥ < 1,6·10³ - met | | | | | | | | | |

**Table 9**

| test strain | validation test | | | | microbial suspension in the test | | test procedure at product concentration V/V | | |
|---|---|---|---|---|---|---|---|---|---|
| | bacterial suspension | Test conditions | neutralizer control | dilution-neutralization control | | | | | |
| | | | | | | | 10% | 50% | 80% |
| | | | | | 10⁻⁶: >330;>330 | Vc . | 0;0 | 0;0 | 0;2 |
| Staphylococcus aureus | Vc: 127;145 | Vc: 134;158 | Vc: 113;120 | Vc: 125;132 | 10⁻⁷: 42;54 | Na | < 140 | < 140 | < 140 |
| ATCC 33592 | Nv₀: 1,4•10² | A: 1,5•10² | B: 1,2•10² | C: 1,3•10² | N: 4,8•10⁸ | lg Na | < 2,15 | < 2,15 | <2,15 |
| | | | | | N₀ 4,8•10⁷ | R | > 5,53 | > 5,53 | > 5,53 |
| | | | | | lgN₀=7,68 | | | | |
| | | | | | 10⁻⁶: >330;>330 | Vc | 0;0 | 0;0 | 0;0 |
| Salmonella enterica sb. enterica | Vc: 102;115 | Vc: 84;108 | Vc: 121;127 | Vc: 98;114 | 10⁻⁷: 39;45 | Na | < 140 | < 140 | < 140 |
| PCM 2565 = ATCC 13311 | Nv₀: 1,1•10² | A: 9,6•10¹ | B: 1,2•10² | C: 1,1•10² | N: 4,2•10⁸ | lg Na | < 2,15 | < 2,15 | < 2,15 |
| | | | | | N₀ 4,2•10⁷ | R | > 5,47 | > 5,47 | > 5,47 |
| | | | | | lgN₀=7,62 | | | | |
| | | | | | 10⁻⁶: 227;251 | Vc | >330;>330 | 0;0 | 0;0 |
| Klebsiella pneumoniae | Vc: 59;74 | Vc: 54;68 | Vc: 71;79 | Vc: 61;64 | 10⁻⁷:25;28 | Na | > 3300 | < 140 | <140 |
| ATCC BAA - 2146 | Nv₀: 6,6•10¹ | A: 6,1•10¹ | B: 7,5•10¹ | C: 6,2•10¹ | N: 2,4•10⁸ | lg Na | > 3,52 | <2,15 | <2,15 |
| | | | | | N₀ 2,4•10⁷ | R | < 3,86 | > 5,23 | > 5,23 |
| | | | | | lgN₀=7,38 | | | | |

| | | | | | | | | | |
|---|---|---|---|---|---|---|---|---|---|
| Vc - viable count Na - cfu/ml in the test mixture N - cfu/ml in the bacterial test suspension, N₀= N/10 A - cfu/ml in the experimental conditions control test Nv - cfu/ml in the bacterial suspension in validation procedure, Nv₀= Nv/10 B - cfu/ml in neutralizer control test R - reduction factor of viable counts C - cfu/ml in the dilution - neutralization method control test Validation criteria verification: 1,5 · 10⁶ < N < 5,0 ·10⁸ - met A,B,C > 0,5 Nv₀ - met 3,0 ·10² < Nᵥ < 1,6 ·10³ - met | | | | | | | | | |

### Antimicrobial Efficacy (VII) - EN1276 test:

The antimicrobial efficacy of example composition E11 at three different dilutions (10% v/v, 50% v/v and 80% v/v, in distilled water) was evaluated in accordance with the EN1276 Standard Suspension Test for bactericidal activity against *Salmonella enterica* (ATCC #13311), *Staphylococcus aureus* (ATCC #33592), *Staphylococcus aureus* (ATCC #6538), *Klebsiella pneumoniae* (ATCC BAA 2146), *Pseudomonas aeruginosa* (ATCC# 15442), *Escherichia coli* (ATCC #10536) and *Enterococcus hirae* (ATCC #10541). Samples of each test composition were placed in test tube with 3% BSA (dirty conditions) and challenge organism to achieve the indicated dilutions for a contact time of 1 minute, followed by neutralization, serial dilutions, plating and incubation. The test was performed at room temperature (approx. 20°C +/- 0.6 °C).The results of the tested compositions are reported on the following Tables 10 and 11.

**Table 10**

| test strain | validation test | | | | microbial suspension in the test | | test procedure at product concentration V/V | | |
|---|---|---|---|---|---|---|---|---|---|
| | bacterial suspension | Test conditions | neutralizer control | dilution-neutralization control | | | | | |
| | | | | | | | 10 % | 50 % | 80 % |
| | Vc: 115;139 | Vc: 119,126 | Vc: 108;120 | Vc: 104;128 | 10⁻⁶: >330;>330 | Vc | >330;>330 | 0;0 | 0;0 |
| Pseudomonas aeruginosa | Nv₀: 1,3•10² | A: 1,2•10² | B: 1,1•10² | C: 1,2•10² | 10⁻⁷: 42;55 | Na | >3300 | < 140 | <140 |
| PCM 2563 = | | | | | N: 4,8•10⁸ | lg Na | > 3,52 | <2,15 | < 2,15 |
| ATCC 15442 | | | | | N₀ 4,8•10⁷ | R | < 4,16 | > 5,53 | > 5,53 |
| | | | | | lgN₀=7,68 | | | | |
| | Vc: 103;110 | Vc: 127;144 | Vc: 98;114 | Vc: 112;125 | 10⁻⁶: >330;>330 | Vc | 0;4 | 13;17 | 6;9 |
| Staphylococcus aureus | Nv₀: 1,1•10² | A: 1,4•10² | B: 1,1•10² | C: 1,2•10² | 10⁻⁷: 39;46 | Na | < 140 | 150 | < 140 |
| ATCC 6538 | | | | | N: 4,2•10⁸ | lgNa | <2,15 | 2,18 | < 2,15 |
| | | | | | N₀ 4,2•10⁷ | R | > 5,47 | 5,44 | > 5,47 |
| | | | | | lgN₀=7,62 | | | | |
| | Vc: 65;78 | Vc: 68;13 | Vc: 57;70 | Vc: 49;54 | 10⁻⁶:254;271 | Vc | 0;0 | 0;1 | 0;0 |
| Escherichia coli | Nv₀: 7,2•10¹ | A: 7,0-10¹ | B: 6,4•10¹ | C: 5,2•10¹ | 10⁻¹:30;36 | Na | < 140 | <140 | <140 |
| PCM 1144 = | | | | | N: 2,7•10⁸ | lg Na | < 2,15 | <2,15 | < 2,15 |
| ATCC 10536 | | | | | N₀ 2,7•10⁷ | R | > 5,28 | > 5,28 | > 5,28 |
| | | | | | lgN₀=7,43 | | | | |
| | Vc: 42;56 | Vc: 38;46 | Vc: 48;53 | Vc: 35;51 | 10⁻⁶: 174;198 | Vc | 0;0 | 0;0 | 0;0 |
| Enterococcus hirae | Nv₀: 4,9•10¹ | A: 4,2•10¹ | B: 5,0•10¹ | C: 4,3•10¹ | 10⁻⁷: 12;19 | Na | < 140 | <140 | < 140 |
| PCM 2559 = | | | | | N: 1,9•10⁸ | lgNa | < 2,15 | <2,15 | < 2,15 |
| ATCC 10541 | | | | | N₀ 1,9•10⁷ | R | > 5,13 | > 5,13 | > 5,13 |
| | | | | | lgN₀=7,28 | | | | |

| | | | | | | | | | |
|---|---|---|---|---|---|---|---|---|---|
| Vc - viable count Na - cfu/ml in the test mixture N-cfu/ml in the bacterial test suspension, N₀= N/10 A - cfu/ml in the experimental conditions control test Nv - cfu/ml in the bacterial suspension in validation procedure, Nv₀= Nv/10 B - cfu/ml in neutralizer control test R - reduction factor of viable counts C - cfu/ml in the dilution - neutralization method control test Validation criteria verification: 1,5·10⁸ < N < 5,0·10⁸ - met A,B,C > 0,5 Nv₀ - met 3,0·10² < Nᵥ < 1,6·10³ - met | | | | | | | | | |

**Table 11**

| test strain | validation test | | | | microbial suspension in the test | | test procedure at product concentration V/V | | |
|---|---|---|---|---|---|---|---|---|---|
| | bacterial suspension | Test conditions | neutralizer control | dilution-neutralization control | | | | | |
| | | | | | | | 10% | 50% | 80% |
| | | | | | 10⁻⁶: >330;>330 | Vc | **0;0** | 3,5 | 0;0 |
| Staphylococcus aureus | Vc: 127;145 | Vc: 134;158 | Vc: 113;120 | V_{c}: 106; 109 | 10⁻⁷: 42;54 | Na | **< 140** | < 140 | < 140 |
| ATCC 33592 | Nv₀: 1,4·10² | A: 1,5·10² | B: 1,2·10² | C: 1,1·10² | N: 4,8·10⁸ | lg Na | **< 2,15** | < 2,15 | < 2,15 |
| | | | | | N₀ 4,8·10⁷ | R | > 5,53 | > 5,53 | > 5,53 |
| | | | | | lg N₀=7,68 | | | | |
| | | | | | 10⁻⁶: >330;>330 | Vc | 0,0 | 0;0 | 0;0 |
| Salmonella enterica sb. enterica | Vc: 102;115 | Vc: 84;108 | Vc. 121;127 | Vc: 117;135 | 10⁻⁷: 39;45 | Na | < 140 | < 140 | < 140 |
| PCM 2565 = ATCC 13311 | Nv₀: 1,1·10² | A: 9,6·10¹ | B: 1,2·10² | C: 1,3·10² | N: 4,2·10⁸ | lg Na | < 2,15 | < 2,15 | < 2,15 |
| | | | | | N₀ 4,2·10⁷ | R | > 5,47 | > 5,47 | > 5,47 |
| | | | | | lgN₀=7,62 | | | | |
| | | | | | 10⁻⁶; 227;251 | Vc | >330;>330 | 0;0 | 0;0 |
| Klebsiella pneumoniae | Vc: 59;74 | Vc: 54;68 | Vc: 71;79 | Vc: 67;80 | 10⁻⁷: 25;28 | Na | > 3300 | < 140 | < 140 |
| ATCC BAA - 2146 | Nv₀: 6,6·10¹ | A: 6,1·10¹ | B: 7,5·10¹ | C: 7,4·10¹ | N: 2,4·10⁸ | lg Na | > 3,52 | < 2,15 | < 2,15 |
| | | | | | N₀ 2,4·10⁷ | R | < 3,86 | > 5,23 | > 5,23 |
| | | | | | lgN₀=7,38 | | | | |

| | | | | | | | | | |
|---|---|---|---|---|---|---|---|---|---|
| **Vc - viable count Na - cfu/mL in the test mixture** **N - cfu/ml in the bacterial test suspension, N₀= N/10 A - cfu/ml In the experimental conditions control test** **Nv - cfu/ml In the bacterial suspension in validation procedure, Nv₀= Nv/10 B - cfu/ml in neutralizer control test** **R- reduction factor of viable counts C - cfu/ml in the dilution - neutralization method control test** **Validation criteria verification:** **1,5·10⁸ < N < 5,0 -10⁸ - met A,B,C 0,5 Nv₀ - met** **3,0 ·10²< Nᵥ < 1,6 ·10³ - met** | | | | | | | | | |

### Antimicrobial Efficacy (VIII) - EN1276 test:

The antimicrobial efficacy of example composition E12 at three different dilutions (10% v/v, 50% v/v and 80% v/v, in distilled water) was evaluated in accordance with the EN1276 Standard Suspension Test for bactericidal activity against *Salmonella enterica* (ATCC #13311), *Staphylococcus aureus* (ATCC #33592), *Staphylococcus aureus* (ATCC #6538), *Klebsiella pneumoniae* (ATCC BAA 2146), *Pseudomonas aeruginosa* (ATCC# 15442), *Escherichia coli* (ATCC #10536) and *Enterococcus hirae* (ATCC #10541). Samples of each test composition were placed in test tube with 3% BSA (dirty conditions) and challenge organism to achieve the indicated dilutions for a contact time of 1 minute, followed by neutralization, serial dilutions, plating and incubation. The test was performed at room temperature (approx. 20°C +/- 0.6 °C).The results of the tested compositions are reported on the following Tables 12 and 13.

**Table 12**

| test strain | validation test | | | | microbial suspension in the test | | test procedure at product concentration V/V | | |
|---|---|---|---|---|---|---|---|---|---|
| | bacterial suspension | Test conditions | neutralizer control | dilution-neutralization control | | | | | |
| | | | | | | | 10% | 50% | 80% |
| | Vc; 115;139 | Vc: 119; 126 | Vc: 108;120 | Vc: 93;98 | 10⁻⁶; >330;>330 | Vc | >330;>330 | 0;0 | 0;0 |
| Pseudomonas aeruginosa | Nv₀: 1,3·10² | A: 1,2·10² | B: 1,1·10² | C: 9,6·10⁸ | 10⁻⁷ 42;55 | Na | > 3300 | < 140 | < 140 |
| PCM 2563 = | | | | | N: 4,8·10⁸ | lg Na | > 3,52 | < 2,15 | < 2,15 |
| ATCC 15442 | | | | | N₀ 4,8·10⁷ | RR | < 4,16 | > 5,53 | > 5,53 |
| | | | | | lgN₀=7,68 | | | | |
| | Vc: 103; 110 | Vc: 127;144 | Vc: 98;114 | Vc:71;84 | 10⁻⁶: >330;>330 | Vc | 1;6 | 32;35 | 23;31 |
| Staphylococcus aureus | Nv₀: 1,1·10² | A: 1,4·10² | B: 1,1·10² | C: 7,8·10¹ | 10⁻⁷: 39;46 | Na | < 140 | 335 | 270 |
| ATCC 6538 | | | | | N: 4,2·10⁸ | lg Na | < 2,15 | 2,53 | 2,43 |
| | | | | | N₀ 4,2·10⁷ | R | > 5,47 | 5,09 | 5,19 |
| | | | | | lgN₀=7.62 | | | | |
| | Vc: 65;78 | Vc:68;73 | Vc: 57;70 | Vc: 53;61 | 10⁻⁶: 254;271 | Vc | 0;0 | 0;0 | 0;0 |
| Escherichia coli | Nv₀: 7,2·10¹ | A: 7,0·10¹ | B: 6,4·10¹ | C: 5,7·10¹ | 10⁻⁷:30;36 | Na | < 140 | < 140 | < 140 |
| PCM 1144= | | | | | N: 2,7·10⁸ | lg Na | < 2,15 | < 2,15 | < 2,15 |
| ATCC 10536 | | | | | N₀ 2,7·10⁷ | R | > 5,28 | > 5,28 | > 5,28 |
| | | | | | IgN₀=7,43 | | | | |
| | Vc: 42;56 | Vc: 38;46 | Vc: 48;53 | Vc: 35;47 | 10⁻⁶: 174;198 | Vc | 0;0 | 0;0 | 0;0 |
| Enterococcus hirae | Nv₀: 4,9·10¹ | A: 4,2·10¹ | B: 5,0·10¹ | C: 4,1·10¹ | 10⁻⁷: 12;19 | Na | < 140 | < 140 | < 140 |
| PCM 2559 = | | | | | N: 1,9·10⁸ | lg Na | < 2,15 | < 2,15 | < 2,15 |
| ATCC 10541 | | | | | N₀ 1,9·10⁷ | R | > 5,13 | > 5,13 | > 5,13 |
| | | | | | lgN₀=7,28 | | | | |

| | | | | | | | | | |
|---|---|---|---|---|---|---|---|---|---|
| **Vc - viable count Na** - **cfu/ml in the test mixture** **N - cfu/ml in the bacterial test suspension, N₀= N/10 A - cfu/mL in the experimental conditions control test** **Nv - cfu/ml in the bacterial suspension in validation procedure, Nv₀= Nv/10 B - cfu/ml in neutralizer control test** **R- reduction factor of viable counts C - cfu/mL in the dilution - neutralization method control test** **Validation criteria verification:** **1,5 ·10⁸<N < 5,0 ·10⁸** - **met A,B,C > 0,5 Nv₀** - **met** **3,0 ·10²< Nᵥ < 1,6 ·10³** - **met** | | | | | | | | | |

**Table 13**

| test strain | validation test | | | | microbial suspension in the test | | test procedure at product concentration V/V | | |
|---|---|---|---|---|---|---|---|---|---|
| | bacterial suspension | Test conditions | neutralizer control | dilution-neutralization control | | | | | |
| | | | | | | | 10% | 50% | 80% |
| | | | | | 10⁻⁶: >330;>330 | Vc | 4;6 | 0;0 | 1;1 |
| Staphylococcus aureus | Vc: 127;145 | Vc: 134;158 | Vc: 113;120 | Vc: 96;101 | 10⁻⁷: 42;54 | Na | < 140 | < 140 | < 140 |
| ATCC 33592 | Nv₀: 1,4·10² | A: 1,5·10² | B: 1,2·10² | C: 9,8·10¹ | N: 4,8·10⁸ | lgNa | < 2,15 | < 2,15 | < 2,15 |
| | | | | | N₀ 4,8·10⁷ | R | > 5,53 | > 5,53 | > 5,53 |
| | | | | | lgN₀=7,68 | | | | |
| | | | | | 10⁻⁶: >330;>330 | Vc | 0;0 | 0;0 | 0;0 |
| Salmonella enterica sb. enterica | Vc: 102;115 | Vc: 84;108 | Vc: 121;127 | Vc: 81;93 | 10⁻⁷: 39;45 | Na | < 140 | < 140 | < 140 |
| PCM2565 = ATCC 13311 | Nv₀: 1,1·10² | A: 9,6·10¹ | B: 1,2·10² | C: 8,7·10¹ | N: 4,2·10⁸ | lgNa | < 2,15 | <2,15 | < 2,15 |
| | | | | | N₀ 4,2·10⁷ | R | > 5,47 | > 5,47 | > 5,47 |
| | | | | | lgN₀=7,62 | | | | |
| | | | | | 10⁻⁶: 227;251 | Vc | >330;>330 | 0;0 | 0;0 |
| Klebsiella pneumoniae | Vc: 59;74 | Vc: 54;68 | Vc: 71;79 | Vc: 52;55 | 10⁻⁷; 25;28 | Na | > 3300 | < 140 | < 140 |
| ATCC BAA - 2146 | Nv₀: 6,6·10¹ | A: 6,1·10¹ | B: 7,5·10¹ | C: 5,4·10¹ | N: 2,4·10⁸ | lg Na | > 3,52 | < 2,15 | < 2,15 |
| | | | | | N₀ 2,4·10⁷ | R | < 3,86 | > 5,23 | > 5,23 |
| | | | | | lgN₀=7,38 | | | | |

| | | | | | | | | | |
|---|---|---|---|---|---|---|---|---|---|
| **Vc - viable count Na - cfu/ml in the test mixture** **N - cfu/ml in the bacterial test suspension, N₀= N/10 A - cfu/ml in the experimental conditions control test** **Nv - cfu/ml in the bacterial suspension in validation procedure, Nv₀= Nv/10 B - cfu/ml in neutralizer control test** **R- reduction factor of viable counts C - cfu/ml in the dilution - neutralization method control test** **Validation criteria verification:** **1,5·10⁸< N < 5,0·10⁸** - **met A,B,C > 0,5 Nv₀** - **met** **3,0·10² < Nᵥ, < 1,6·10³** - **met** | | | | | | | | | |

### Antimicrobial Efficacy (IX) - Handwash Test:

The antimicrobial efficacy of example composition E11 of Table 1 was evaluated as to its efficacy in the reduction of a challenge microorganism in accordance with the test protocls of EN 1499 (:2000) "Hygienic Handwash". The test utilized 15 human subjects each having a left and right hand. The antimicrobial performance of the E11 composition was compared to that of a reference 'soft soap' (having a density of 200 g/litre) as outlined in EN 1449. The E11 compositions were tested without further dilution and evaluated for antimicrobial performance against the challenge microorganism *Escherichia coli* (PCM 2560 = NCTC 10538). The test was performed at room temperature and all materials used in the test were also at room temperature (20°C +/- 0.6°C). An aliquot (3 ml) of undiluted E11 was applied to the subject's hands, and the handwash protocols of EN 1449 - Appendix A handwashing continued for 30 seconds, after which the subject rinsed their hands for 15 seconds under a stream of tap water. The test results are reported below in the following Table 14 which reports the relative performance of the E11 composition applied as indicated above, against the reference 'soft soap' which was however applied in an amount of 5 ml, onto wet hands and handwashing continued for 60 seconds prior to rinsing in a stream of tap water for 15 seconds., thus having double the contact time with the hands than the E1 composition.

**Table 14**

| person | (comparative) | | | (example composition) | | |
|---|---|---|---|---|---|---|
| | 'soft soap' | | | E11 | | |
| | Log x | Log y | Log z | Log x | Log y | Log z |
| 1 | 7,02 | 3,97 | 3,05 | 6,66 | 3,51 | 3,15 |
| 2 | 6,77 | 3,65 | 3,12 | 6,98 | 3,30 | 3,68 |
| 3 | 6,51 | 3,73 | 2,78 | 6,30 | 3,36 | 2,94 |
| 4 | 6,96 | 3,60 | 3,36 | 6,80 | 3,13 | 3,67 |
| 5 | 6,84 | 3,49 | 3,35 | 6,87 | 3,08 | 3,79 |
| 6 | 6,37 | 3,78 | 2,59 | 6,70 | 3.76 | 2,94 |
| 7 | 6,22 | 3,07 | 3,15 | 6,22 | 2,99 | 3,23 |
| 8 | 6,99 | 3,72 | 3,27 | 6,83 | 3,06 | 3,77 |
| 9 | 6,01 | 3,40 | 2,61 | 6,36 | 3,00 | 3,36 |
| 10 | 6,87 | 3,74 | 3,13 | 7,04 | 3,64 | 3,40 |
| 11 | 6,91 | 3,62 | 3,29 | 6,78 | 3,09 | 3,69 |
| 12 | 7,04 | 3,94 | 3,10 | 6,80 | 3,09 | 3,71 |
| 13 | 6,33 | 3,46 | 2,87 | 6,29 | 2,94 | 3,35 |
| 14 | 6,64 | 3,66 | 2,98 | 6,96 | 2,93 | 4,03 |
| 15 | 6,73 | 3,54 | 3,19 | 6,74 | 2,91 | 3,83 |
| X(m) | 6,68 | 3,62 | 3,06 | 6,69 | 3,19 | 3,50 |
| S | 0,32 | 0,22 | 0,25 | 0,27 | 0,27 | 0,33 |
| S' | 0,09 | 0,06 | 0,07 | 0,07 | 0,07 | 0,09 |
| N | 15 | 15 | 15 | 15 | 15 | 15 |
| Log x = log of pre-count value / 1 ml TSB | | | | | | |
| Log y = log of post-count value / 1 ml TSB | | | | | | |
| Log z = log of reduction factor | | | | | | |
| X(m) = mean | | | | | | |
| S = standard deviation | | | | | | |
| S'= standard deviation of the mean | | | | | | |
| N = number of subjects in the test | | | | | | |

As is evident from the reported results of Table 14, the mean Log₁₀ reduction of the challenge microorganism (reported as "Log z" on Table 14) was superior to that of the mean Log₁₀ reduction of the 'soft soap'.

While the invention is susceptible of various modifications and alternative forms, it is to be understood that specific embodiments thereof have been shown by way of example which are not intended to limit the invention to the particular forms disclosed; on the contrary the intention is to cover all modifications, equivalents and alternatives falling within the scope and spirit of the invention as expressed in the appended claims.

## Claims

1. A highly aqueous, antimicrobially effective topical compositions which comprises:
at least one first cationic surfactant or salt thereof having antimicrobial or germicidal properties or salt form thereof;
at least one different, second quaternary ammonium cationic surfactant or salt form thereof having antimicrobial or germicidal properties according to the structure (I) 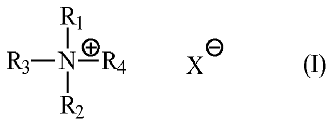
wherein:
R₁ is a C₁₆, preferably an unsubstituted alkyl moiety which may optionally be branched but is preferably unbranched,
R₂, R₃ and R₄ are independently C₁-C₃ alkyl moieties, and,
X is a salt forming counterion which renders the second quaternary surfactant water soluble or water dispersible, and,
an amine oxide nonionic surfactant which is the predominant further surfactant in the composition,
wherein the compositions are in the pH range of 4.5 - 6, and which compositions are low viscosity, pourable unpressurized liquids which are used as cleansing compositions for topical surfaces.

2. A composition according to claim 1, wherein:
the amine oxide surfactant comprises at least 90%, of the total weight of all surfactants present in the composition other than the amounts of the at least one first cationic surfactant having antimicrobial or germicidal properties or salt form thereof and with the at least one different, second quaternary ammonium cationic surfactant or salt form thereof.

3. A composition according to claim 1 or 2, wherein the amine oxide surfactant comprises at least 95%, of the total weight of all surfactants present in the composition other than the amounts of the at least one first cationic surfactant having antimicrobial or germicidal properties or salt form thereof and with the at least one different, second quaternary ammonium cationic surfactant or salt form thereof

4. A composition according to any of claims 1 - 3, wherein the amine oxide surfactant is the sole further surfactants present in composition other than the amounts of the at least one first cationic surfactant having antimicrobial or germicidal properties or salt form thereof and with the at least one different, second quaternary ammonium cationic surfactant or salt form thereof.

5. A composition according to any of claims 1 - 4, wherein the compositions are in the pH range of 4.5 - 5.5.

6. A composition according to any of claims 1 - 5, wherein the composition further comprises: glycerin, a glycol ether, and a oxyalkylenated compound.

7. A composition according to claim 6 wherein the oxyalkylenated compound is a polyethylene glycol having from 100 to 200 ethylene oxide units.

8. A composition according to any of claims 1 - 7, wherein the said composition exhibits at least a 3 x log₁₀ reduction (99.9%), preferably at least a 4 x log₁₀ reduction of one or more of the challenge microorganisms selected from one or more, preferably at least two or more, ore preferably at least 3 or more of the following microorganisms: *E. coli, S. aureus, P. aeruginosa,* and *E. hirae* according to EN 1276.

9. A composition according to any of claims 1 - 7, wherein the said composition exhibits at least a 4 x log₁₀ reduction of the following challenge microorganisms:
*Enterococcus faecalis* (ATCC #51299)
*Esherichia coli* (ATCC #11229)
*Klebsiella pneumoniae pneumoniae* (ATCC # 10031)
*Psuedomonas aeruginosa* (ATCC #9027)
*Staphylococcus aureus aureus* (ATCC #6538)
*Staphylococcus aureus aureus* MRSA(ATCC #33592)
*Staphylococcus epidermidis* (ATCC #12228)
*Streptococcus pneumoniae* (ATCC #6303)
*Streptococcus pyogenes* (ATCC #19615)
when tested according to ASTM E-1054-08, *Standard Test Methods for Evaluation of Inactivators of Antimicrobial Agents* for a 60 second contact time.

10. A composition according to any of claims 1 - 9, wherein the composition comprises:-
a) 1.4 wt% PEG-150 distearate
b) 0.1 wt% coconut monoethanolamide
c) 0.9 wt% cetrimonium chloride
d) 0.1 wt% benzalkonium chloride
e) 1.5 wt% lauryl dimethyl amide oxide
f) 2.0 wt% glycerin
g) 0.5 wt% propylene glycol
h) 0.2 wt% tetrasodium EDTA
i) 0.23 wt% citric acid

11. A method a method for providing an antimicrobial benefit to a topical surface, other body area or hair, which method includes the step of:
applying an antimicrobially effective amount of a topical germicidal composition according to any preceding claim, to reduce the incidence of undesired microorganisms present on the treated topical surface, other body surface or hair.
